# EUROPEAN PATENT APPLICATION

(11) **EP 4 527 946 A1**
(43) Date of publication of application: **26.03.2025**
(21) Application number: 23382968.8
(22) Date of filing: 25.09.2023
(51) Int. Cl.: C12Q 1/6886

(54) **METHOD FOR PREDICTING THE RESPONSE TO A DUAL SPECIFICITY PROTEIN KINASE TTK INHIBITOR THERAPY**

(71) Applicant: Pangaea Oncology, S.A, 08028 Barcelona (ES)
(72) Inventor: MOLINA VILA, Miguel Ángel, E-08028 Barcelona (ES); BERTRÁN ALAMILLO, Jordi, E-08028 Barcelona (ES)
(74) Representative: ABG Intellectual Property Law, S.L.

(57) **Abstract**

The present invention relates to the field of cancer and, more particularly, to methods for predicting the response of a patient diagnosed with cancer to a dual specificity protein kinase TTK inhibitor (TTKi) therapy based on the detection of biomarkers in a sample of the patient. It also relates to methods for selecting a customized therapy for a patient suffering cancer.

## Description

### FIELD OF THE INVENTION

The present invention relates to the field of cancer and, more particularly, to methods for predicting the response of a patient diagnosed with cancer to a dual specificity protein kinase TTK inhibitor (TTKi) therapy based on the detection of biomarkers in a sample of the patient. It also relates to methods for selecting a customized therapy for a patient suffering cancer.

### BACKGROUND OF THE INVENTION

Targeted therapies bring great benefit to cancer patients because they can improve survival rates with fewer side effects than traditional, less selective cytotoxic drugs. Small molecule inhibitors of protein kinases are a prime example of the success of targeted therapy: many of these inhibitors exploit unique features of tumor cells, permitting cancer specificity while having limited effects on healthy cells. A classic example of a targeted therapy is the use of tyrosine kinase inhibitors and antibodies in breast cancer patients with amplification or overexpression of the HER2 gene.

To determine whether it is likely that a patient will respond to a certain targeted therapy, it is important to determine the status and presence of biomarkers that correlate with drug sensitivity in specimens of the patient's tumor, before the start of treatment.

Targeted therapies, where antitumor drugs are selected based on specific biomarkers, have revolutionized the treatment of some malignancies. Examples include tyrosine kinase inhibitors (TKIs) for non-small cell lung cancer (NSCLC) patients with mutations in the Epidermal Growth Factor Receptor (EGFR-mut), poly [ADP-ribose] polymerase 1 (PARP1) inhibitors for tumors defective in DNA repair or anti-receptor tyrosine-protein kinase erbB-2 (ERBB2) antibodies for ERBB2-amplified breast cancer. However, most cancer patients are pan-negative for known, clinically relevant biomarkers and are thus ineligible for targeted therapy. Conversely, a significant number of new drugs lack adequate markers for patient selection.

Several dual specificity protein kinase TTK inhibitor (TTKi) are currently in clinical trials in different malignancies and partial responses have been observed. However, the clinical application of TTKi has been hampered by several obstacles. First, TTK mRNA or protein levels do not correlate with response and there are currently no predictive markers for patient selection. Second, it is known that TTKi leads to abnormal segregation of chromosomes and polyploidy/aneuploidy, but the cell and molecular responses subsequently triggered are poorly understood. In particular, some tumor cells experience cycle arrest and survival and others cell death after spindle assembly checkpoint (SAC) abrogation due to factors so far unidentified.

The spindle assembly checkpoint (SAC) plays a pivotal role in the control of mitosis and drugs targeting SAC, particularly dual specific protein kinase TTK, are in different stages of clinical development. However, cell response to TTK inhibitors (TTKis) is poorly understood and there are no markers for patient selection. Tumor cells show a dual response to TTKis, with some cells undergoing apoptosis and others experiencing cell cycle arrest, senescence and survival. Moreover, different cancer cell lines show different relative sensitivities for TTKis.

Therefore, in view of the broad activity of TTKi in many different cell lines and tumor types, there is a clear need for biomarkers that can be used to predict which cancers are most likely to respond to chemotherapeutic treatment with a TTKi.

### SUMMARY OF THE INVENTION

The authors of the present invention have demonstrate that the dual response to a dual specificity protein kinase TTK inhibitor (TTKi) is regulated by the levels of BH3-interacting domain death agonist (BID). Overexpression of BID switches cancer cells from survival to death after TTKi and, consequently, associates with sensitivity to TTKi.

Thus, in a first aspect, the present invention relates to an *in vitro* method for predicting the response of a patient suffering cancer to a dual specificity protein kinase TTK inhibitor (TTKi) therapy that comprises:
i) determining the expression level of BH3-interacting domain death agonist (BID) in a sample from said patient and,
ii) comparing the expression level obtained in (i) to a reference value,
wherein if the expression level of BID in a sample from the patient is above a reference value, it indicates that the patient is predicted to respond to the TTKi therapy.

In another aspect, the present invention relates to an *in vitro* method for designing a personalized therapy for a patient suffering cancer that comprises:
i) determining the expression level of BH3-interacting domain death agonist (BID) in a sample from said patient and,
ii) comparing the expression level obtained in (i) to a reference value,
wherein a expression level of BID above a reference value is indicative that the patient is to be treated with a TTKi.

In another aspect, the present invention relates to a pharmaceutical composition, hereainafter the pharmaceutical composition of the invention, comprising a TTKi, a BH3 mimetic agent and at least one pharmaceutically acceptable excipient.

In another aspect, the present invention relates to the pharmaceutical composition of the invention for use in the treatment of cancer.

In another aspect, the present invention relates to the use of reagents specific for the determination of the expression level of BID for predicting the response of a patient suffering from cancer to a dual specificity protein kinase TTK inhibitor (TTKi) therapy or for designing a personalized therapy for a patient suffering cancer.

In another aspect, the present invention relates to a method for predicting the response of a patient suffering from cancer to a TTKi therapy and treating the patient that comprises:
a) determining the expression level of BH3-interacting domain death agonist (BID) in a sample from said patient; and
b) comparing the expression level obtained in (i) to a reference value,
wherein if the expression level of BID in the sample from the patient is above a reference value, said patient is treated with a TTKi.

### BRIEF DESCRIPTION OF THE DRAWINGS

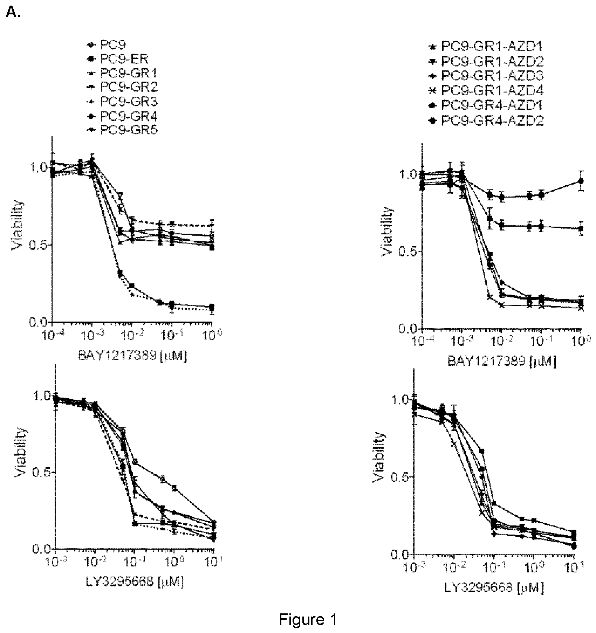

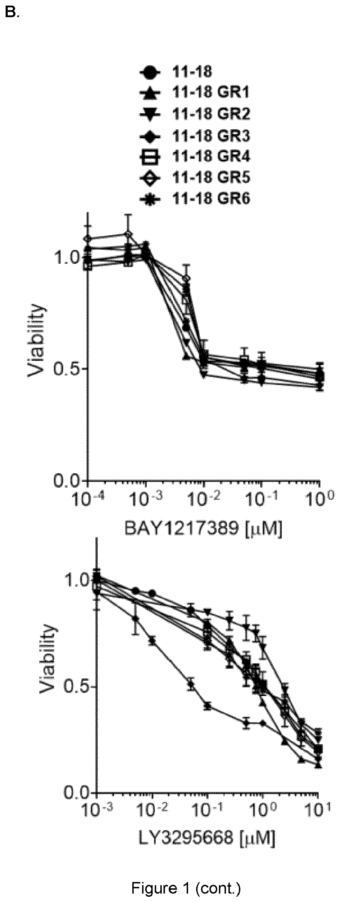

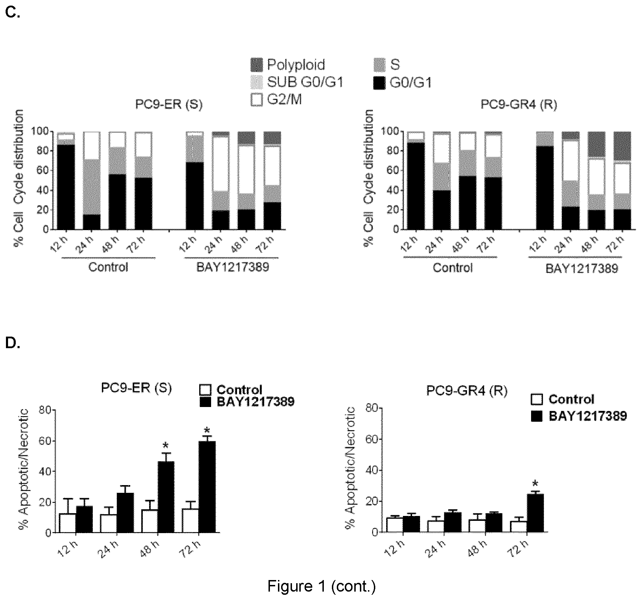

**Figure 1****.** (A) Dose-response plots to BAY1217389 and LY3295668 of EGFR TKI-resistant clones derived from the PC9 cell line. Values shown are means ± SD, experiments were conducted in triplicates (n=3). In each experiment, every concentration of drug was tested in sextuplicates (n = 6). **(B)** Dose-response plots to BAY1217389 and LY3295668 of EGFR TKI resistant clones derived from 11-18 cells. **(C)** Cell cycle analysis of PC9-ER and PC-GR4 clones. Cells were serum-starved for 24 h, FBS (10%) and BAY1217389 (50 nM) were added and cultures submitted to PI staining at the indicated times. **(D)** Percentage of apoptotic/necrotic cells in PC9-ER and PC-GR4 cultures. Cells were serum-starved for 24 h, FBS (10% final) and BAY1217389 (50 nM) were added and cultures submitted to annexin V/propidium iodide staining at the indicated times. Values shown are means ± SD, experiments were conducted in duplicates (n=2).

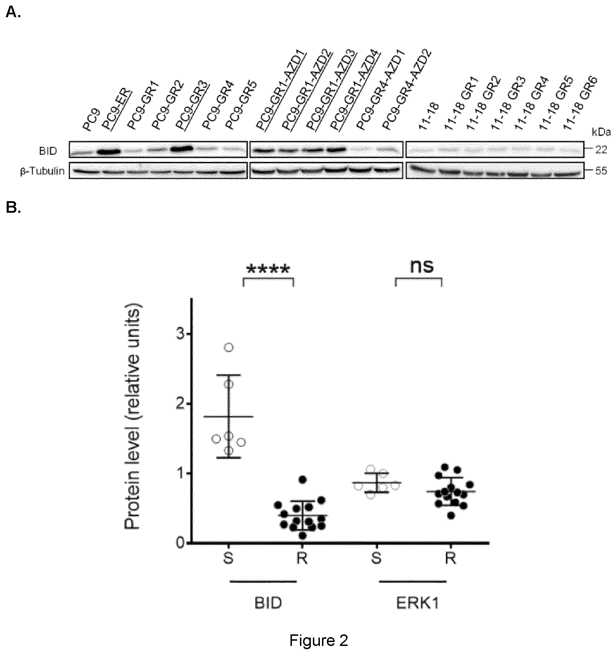

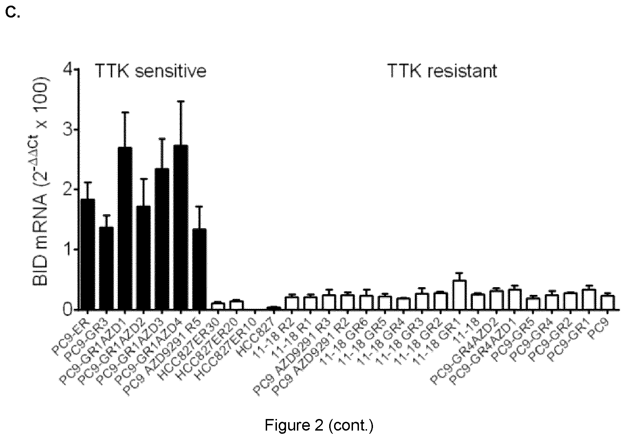

**Figure 2**. (A) Western blotting analysis of BID in PC9 and 11-18-derived clones. Underlined lines are sensitive to TTKi. **(B)** Levels of 2 proteins (BID, and Erk1) in PC9 and 11-18 derived clones, classified according to sensitivity to TTKi (S=sensitive, R=resistant). Erk1 was included as a control. Each point represents a clone, lines. indicate mean ± SD. ***p<0.001, **p<0.001, *p<0.05; ns, not significant (Student's t test (C) BID mRNA levels in individual PC9 and 11-18 derived clones, as determined by RT-Q-PCR. Data were normalized against beta-actin (mean ± SD, n>3).

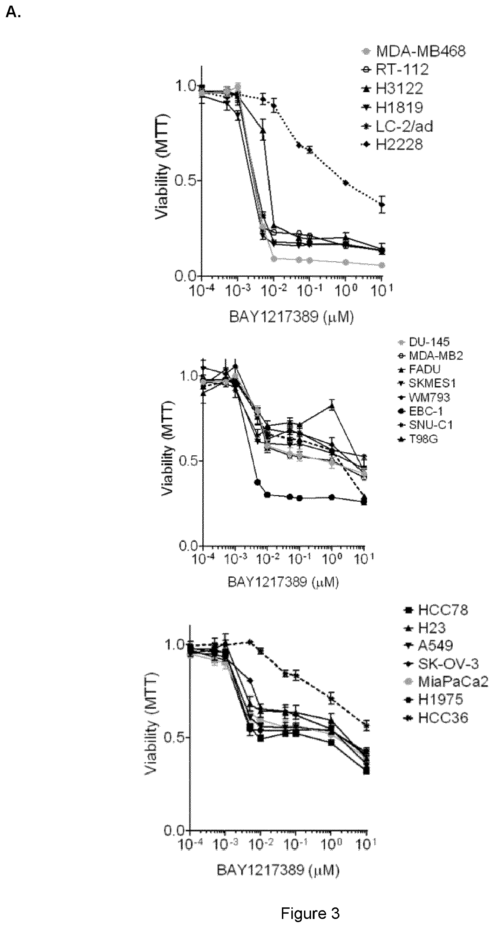

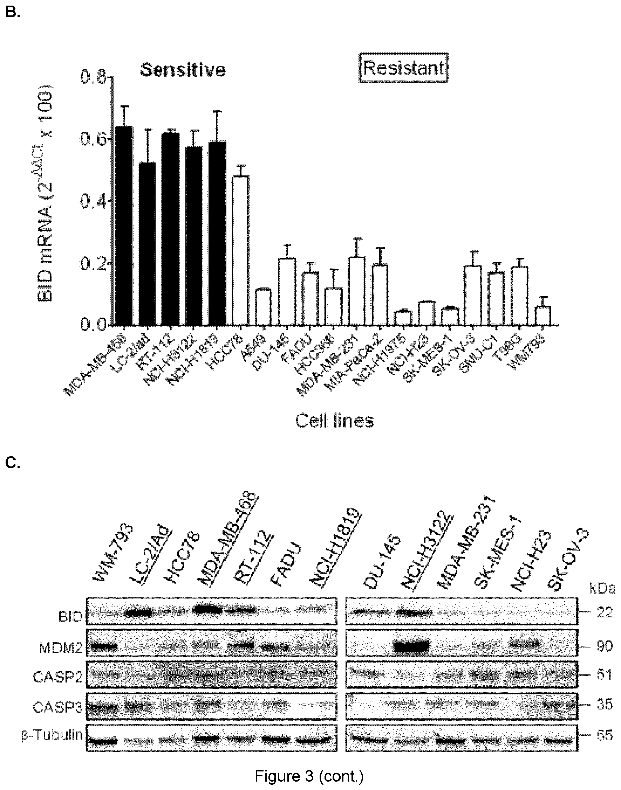

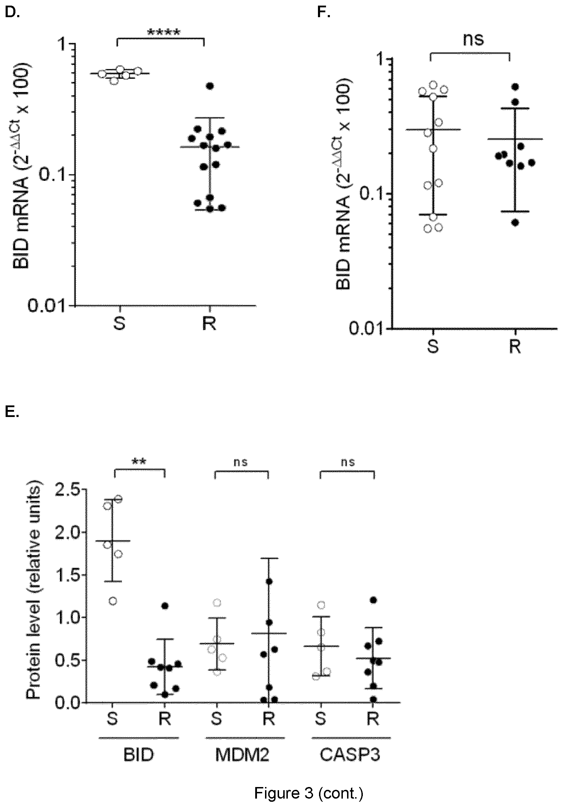

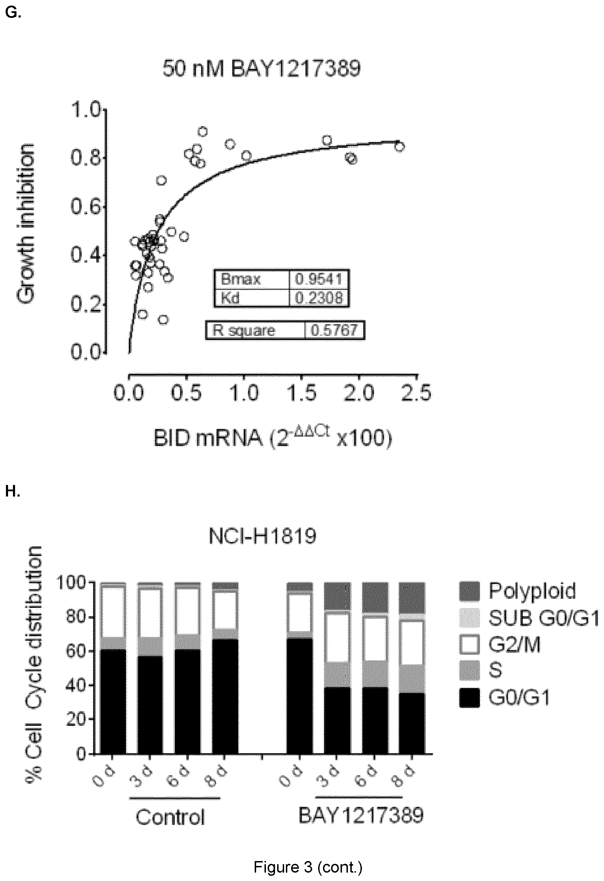

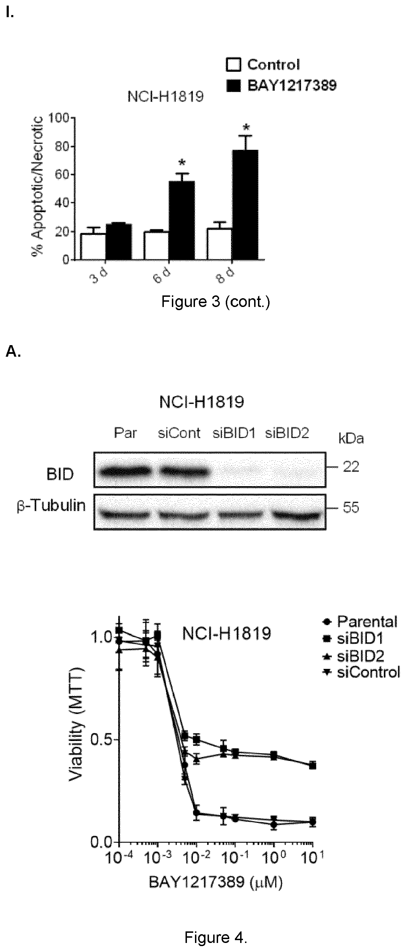

**Figure 3****.** (A) Dose-response plots to BAY1217389 of cancer cell lines derived from lung, prostate, breast, pancreas, ovarian, colon and other tumors. Left panel, cell lines sensitive to BAY1217389; middle and right panels, cell lines resistant to BAY1217389. Values shown are means ± SD, experiments were conducted at least in duplicates (n>2). In each experiment, every concentration of drug was tested in sextuplicates (n = 6). **(B)** RT-Q-PCR analysis of BID mRNA levels in cancer cell lines derived from lung, prostate, breast, pancreas, ovarian, colon and other tumors. Data were normalized against beta-actin (mean ± SD, n>3). Lines sensitive and resistant to TTKi are indicated in white and black, respectively. **(C)** Western blotting analysis of BID, MDM2 and CASP-3 protein levels in cancer cell lines derived from lung, prostate, breast, pancreas, ovarian, colon and other tumors. Underlined lines are sensitive to TTKi. **(D)** BID mRNA levels in cancer cell lines of diverse origins, classified according to response to TTKi (S, sensitive; R, resistant). Each point represents a cell line, lines indicate mean ± SD. ****p<0.0001 (Student's t test). **(E)** BID, MDM2 and CASP3 protein levels in cancer cell lines of diverse origins, classified according to sensitivity to TTKi. Each point represents a cell line, lines indicate mean ± SD. **p<0.001; ns=not significant (Student's t test). **(F)** BID mRNA levels in cancer cell lines of diverse origins, classified according to response to LY3295668 (S, sensitive cells, IC50<1 µM; R, resistant cells, IC50>10 µM). Each point represents a cell line, lines indicate mean ± SD. ns= not significant (Student's ttest). **(G)** Plot of BID mRNA levels vs. cell growth inhibition at 50 nM BAY1217389 in EGFR-mut and EGFR-wt cancer cells of different origins. **(H)** Cell cycle analysis of the BAY1217389 -sensitive NCI-H1819 cells at different timepoints. C, control cells; A, cells treated with 50 nM BAY1217389. **(I)** Annexin V analysis of the BAY1217389 -sensitive NCl-H1819 cells at different timepoints

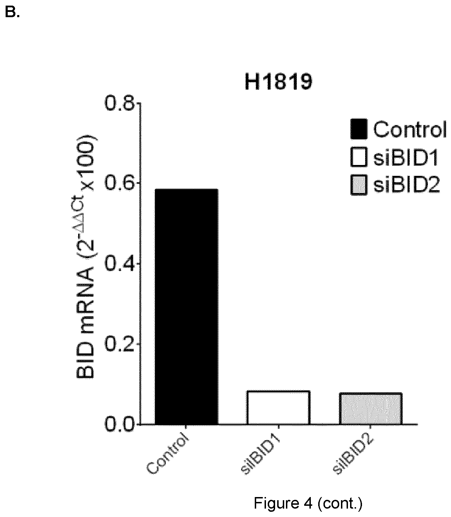

**Figure 4****.** (A) The BAY1217389-sensitive NCl-H1819 cell were transfected with control shRNA lentiviral particles to silence BID. After puromycin selection, colonies were analyzed by Western blotting and the dose response plots to BAY1217389 were obtained for selected colonies. **(B)** Levels of BID mRNA in parental NCI-H1819 cells (black bars) and clones silenced with shRNA lentiviruses (white and grey bars).

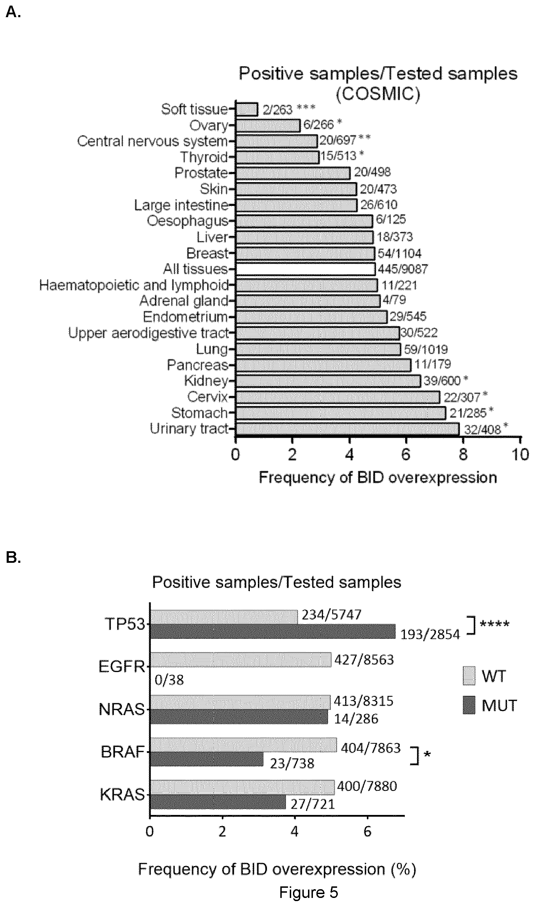

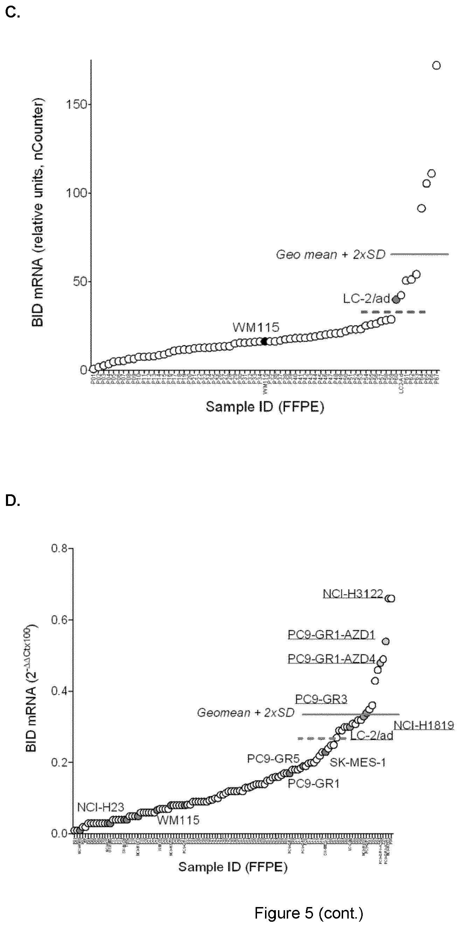

**Figure 5****.** (A) Frequency of BID mRNA upregulation in human malignancies, as found in the COSMIC database. Asterisks denote significant differences with the entire tumor cohort (light bars) ***p<0.001, **p<0.01, *p<0.05 (two-tailed z-score). **(B)** Analysis of co-occurrence of BID overexpression with KRAS, BRAF, NRAS and EGFR activating mutations in the samples of COSMIC database with sequencing results and BID expression data. *p<0.05 (two-tailed z score). **(C)** Levels of BID mRNA, as quantified by nCounter, in FFPE blocks from a cohort of 67 advanced lung tumors. **(D)** Levels of BID mRNA, as quantified by RT-Q-PCR in FFPE blocks from a cohort of 94 tumor samples.

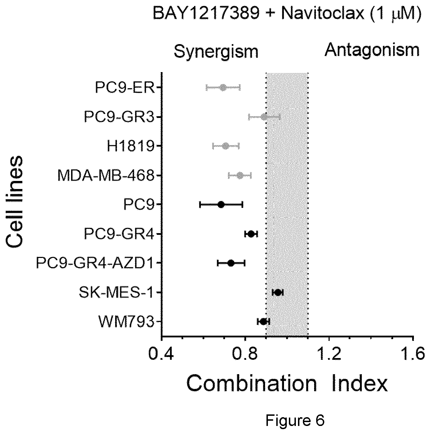

**Figure 6****.** Combination indexes of BAY123789 with navitoclax (1µM). Values shown are means ± SEM of two independent experiments. The blue shaded area indicates the 0.9-1.1 range of the Combination Index (CI), which corresponds to an additive or nearly additive interaction between drugs.

### DETAILED DESCRIPTION OF THE INVENTION

*In vitro method for predicting the response of a patient suffering cancer to a dual specificity protein kinase TTK inhibitor (TTKi) therapy*

The authors of the present invention have demonstrated that the response to a dual specificity protein kinase TTK inhibitor (TTKi) is regulated by the levels of BH3-interacting domain death agonist (BID). Overexpression of BID switches cancer cells from survival to death after TTKi and, consequently, associates with sensitivity to TTKi.

Accordingly, in a first aspect, the present invention relates to an *in vitro* method, hereinafter the first method of the invention, for predicting the response of a patient suffering cancer to a dual specificity protein kinase TTK inhibitor (TTKi) therapy that comprises:
i) determining the expression level of BH3-interacting domain death agonist (BID) in a sample from said patient and,
ii) comparing the expression level obtained in (i) to a reference value, wherein if the expression level of BID in a sample from the patient is above a reference value, it indicates that the patient is predicted to respond to the TTKi therapy.

The term *"in vitro",* as used herein, refers to the fact that an experimental protocol or a method is not carried out in the body of a human or animal subject or patient, but in samples isolated from said subject, and already present in a laboratory tool, such as a test tube, dish or plate.

The term "predicting", as used herein, refers to the determination of the likelihood that the patient suffering from cancer will respond favorably to an anti-cancer therapy, particularly a TTKi therapy. Especially, the term "prediction", as used herein, relates to an individual assessment of the expected response of a patient suffering from cancer if the tumor is treated with a TTKi therapy.

Standard criteria (Eisenhauer, E.A., et al. 2009. New response evaluation criteria in solid tumours: revised RECIST guideline (version 1.1). Eur J Cancer 45(2): 228-247) that can be used herewith to evaluate the response to an anti-cancer therapy include response, stabilization, and progression. The term "RECIST", as used herein, refers to the "Response Evaluation Criteria in Solid Tumors" and it is a standard way to measure how well a cancer patient responds to treatment. It is based on whether tumors shrink, stay the same, or get bigger. To use RECIST, there must be at least one tumor that can be measured on x-rays, computed tomography (CT) scans, or magnetic resonance imaging (MRI) scans. A patient can have a complete response (CR), a partial response (PR), progressive disease (PD), and stable disease (SD).

In the context of the present invention, patients achieving complete or partial response and patients showing a stable disease were considered as "clinical responders" or having a clinical response.

A "complete response" (or complete remission) (CR), as used herein, is the disappearance of all detectable malignant disease, i.e., the disappearance of all target lesions.

A "partial response" (PR) is defined herein as at least a 30% decrease in the sum of diameters of target lesions, taking as reference the baseline sum diameters.

The term "stabilization", "stabilization of the disease" or "stable disease" (SD), as used herein, is considered when there is neither sufficient shrinkage to qualify for partial response nor sufficient increase to qualify for progressive disease, taking as reference the smallest sum diameters while on study.

In the context of the present invention, patients showing progressive disease are considered as "poor responders" or having a poor response.

The term "progression" or "progressive disease" (PD), as used herein, is defined as at least a 20% increase in the sum of diameters of target lesions, taking as reference the smallest sum on study (this includes the baseline sum if that is the smallest on study). In addition to the relative increase of 20%, the sum must also demonstrate an absolute increase of at least 5 mm. The appearance of one or more new lesions is also considered progression.

As the person skilled in the art will understand, having a poor response to the TTKi therapy does not mean that the patient has no response at all or that the treatment has no effect on the patient. For example, the TTKi therapy may reduce the growth of the lesion in a poor responder when compared with a patient that does not undergo any therapy. But the effect achieved in said patient when compared to an untreated patient does not qualify to be considered stabilization under the criteria used in the present invention. For example, a patient treated with a TTKi therapy can have an increase of 20% in the sum of diameters of target lesions, but this increase could have been of 30% if the patient is left untreated.

As will be understood by those skilled in the art, such an assessment is usually not intended to be correct for all (i.e. 100 percent) of the patients to be identified. The term, however, requires that the prediction provides correct results for a statistically significant portion of patients. Whether a portion is statistically significant can be determined without further ado by the person skilled in the art using various well known statistic evaluation tools, e.g., determination of confidence intervals, p-value determination, Student's t-test, Mann-Whitney test, etc. Details are found in Dowdy and Wearden, Statistics for Research, John Wiley and Sons, New York 1983. Preferred confidence intervals are at least 50%, at least 60%, at least 70%, at least 80%, at least 90 percent, at least 95 percent, at least 97 percent, at least 98 percent or at least 99 percent. The p- values are, preferably, 0.1, 0.05, 0.01, 0.005, 0.001, 0.0005, 0.0001 or less. More preferably, at least 60 percent, at least 70 percent, at least 80 percent or at least 90 percent of the patients of a population can be properly identified by the method of the present invention.

Any other parameter which is widely accepted for comparing the efficacy of alternative treatments can be used for determining the response to a treatment and include, without limitation:
- disease-free progression which, as used herein, describes the proportion of patients in complete remission who have had no recurrence of disease during the time period under study.
- disease-free survival (DFS) which, as used herewith, is understood as the length of time after treatment for a disease during which a patient survives with no sign of the disease.
- objective response which, as used in the present invention, describes the proportion of treated patients in whom a complete or partial response is observed.
- tumor control which, as used in the present invention, relates to the proportion of treated patients in whom complete response, partial response, minor response or stable disease ≥ 6 months is observed.
- progression free survival which, as used herein, is defined as the time from start of treatment to the first measurement of cancer growth.
- time to progression (TTP), as used herein, relates to the time after a disease is treated until the disease starts to get worse. The term "progression" has been previously defined.
- six-month progression free survival or "PFS6" rate which, as used herein, relates to the percentage of patients wherein free of progression in the first six months after the initiation of the therapy.
- median survival which, as used herein, relates to the time at which half of the patients enrolled in the study are still alive.
- overall survival which, as used herein, refers to the length of time from either the date of diagnosis or the start of treatment for a disease, such as cancer, that patients diagnosed with the disease are still alive.
- recurrence which, as used herein, refers to the appearance of cancer after treatment and after a period of time during which cancer was not detected.
- survival without metastasis, as used herein, refers to the length of time after treatment for a cancer ends that the patient survives without any signs or symptoms of cancer metastasis.
- decrease in circulating tumor cells, as used herein, refers to a decrease in the concentration of circulating tumor cells in the blood or in the lymph of a patient with metastatic cancer. A decrease in circulating tumor cells is associated with efficacy of a therapy against the metastatic cancer.
- response of circulating markers, as used herein, refers to the variation on the concentration in blood or in the lymph of proteins or nucleic acids associated with a specific cancer after treatment against said specific cancer.

The first method of the invention is carried out in a patient suffering from cancer, i.e., in a patient that has been previously diagnosed with cancer.

The term "patient" or "subject", as used herein, refers to human beings. Preferably, the subject is a male or female human of any age or race. In the context of the present invention, the subject is a subject suffering from cancer or previously diagnosed with cancer. In a preferred embodiment, the subject is a mammal, preferably a human being.

The subjects tested in the first method of the invention have been previously diagnosed with cancer. The term "diagnosed", as used herein, refers to the determination and/or identification of a disease in a subject, i.e., the opinion reached about the disease state of a subject, i.e., the diagnosis opinion. As such, it can also be regarded as an attempt to classify individuals depending on their disease condition. As it will be understood by those skilled in the art, the diagnosis of cancer, although preferred to be, need not be correct for 100% of the subjects to be diagnosed or evaluated. The term, however, requires that a statistically significant portion of subjects identified as such are suffering cancer. Methods to determine if a portion of subjects is statistically significant have been disclosed above in relation to the method of prediction.

The first method of the invention is suitable for any type of cancer. The term "cancer" is referred to a group of diseases characterized by uncontrolled cell division (or by an increase of survival or apoptosis resistance) and by the ability of said cells to invade other neighboring tissues (invasion) and spread to other areas of the body where the cells are not normally located (metastasis) through the lymphatic and blood vessels, circulate through the bloodstream, and then invade normal tissues elsewhere in the body. Depending on whether or not they can spread by invasion and metastasis, tumors are classified as being either benign or malignant: benign tumors are tumors that cannot spread by invasion or metastasis, i.e., they only grow locally; whereas malignant tumors are tumors that are capable of spreading by invasion and metastasis. Biological processes known to be related to cancer include angiogenesis, immune cell infiltration, cell migration and metastasis.

The term "cancer" includes, without limitation, leukemias (e.g., acute leukemia, acute lymphocytic leukemia, acute myelocytic leukemia, acute myeloblastic leukemia, acute promyelocytic leukemia, acute myelomonocytic leukemia, acute monocytic leukemia, acute erythroleukemia, chronic leukemia, chronic myelocytic leukemia, chronic lymphocytic leukemia), hairy cell leukemia, polycythemia vera, lymphoma (e.g., Hodgkin's disease or non-Hodgkin's disease), CNS lymphoma, AIDS-associated leukemias, Waldenstrom's macroglobulinemia, multiple myeloma, heavy chain disease, and solid tumors such as sarcomas and carcinomas (e.g., fibrosarcoma, myxosarcoma, liposarcoma, chondrosarcoma, osteogenic sarcoma, chordoma, angiosarcoma, mendotheliosarcoma, lymphangiosarcoma, lymphangioendotheliosarcoma, synovioma, mesothelioma, Ewing's tumor, leiomyosarcoma, rhabdomyosarcoma, Kaposi's sarcoma, colon carcinoma, pancreatic cancer, lung cancer, colon cancer, colorectal cancer, bladder cancer, breast cancer, biliary tract cancer, esophageal cancer, stomach/gastric cancer, ovarian cancer including those arising from epithelial cells, stromal cells, germ cells and mesenchymal cells; prostate cancer, oral cancer including squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, teratoma, choriocarcinoma, endometrial/uterine/cervical cancer, seminoma, embryonal carcinoma, Wilm's tumor, cervical cancer, uterine cancer, testicular cancer, lung carcinoma, small cell lung carcinoma, bladder carcinoma, epithelial carcinoma, intraepithelial neoplasms including Bowen's disease and Paget's disease, neuroglioma, glioma, mixed glioma, optic nerve glioma, subependymoma, metastatic brain tumor, pituitary tumors, primitive neuroectodermal (PNET) tumor, juvenile pilocytic astrocytoma (JPA), brain stem glioma, astrocytoma, pineal tumor, rhabdoid tumor, glioblastoma multiforme (GBM, also known as glioblastoma), medulloblastoma, craniopharyngioma, ependymoma, pinealoma, hemangioblastoma, acoustic neuroma, oligodendroglioma, schwannoma, neurofibrosarcoma, meningioma, melanoma, neuroblastoma, and retinoblastoma).

The term "cancer" also includes, without limitation, head and neck cancer, leukemia, cancer of the heart, esophageal cancer, cancer of the small intestine, spleen cancer, kidney cancer, brain cancer, choriocarcinoma, skin cancer, bone cancer, bone marrow, blood, thymus, womb, liver cancer, sarcoma, liposarcoma, fibrosarcoma, Merkel cell carcinoma, Kaposi's sarcoma, testicular cancer including germinal tumors such as seminoma, non-seminoma (teratomas, choriocarcinomas), stromal tumors, and germ cell tumors; thyroid cancer including thyroid adenocarcinoma and medullar carcinoma; renal cancer including adenocarcinoma and Wilms tumor; cholangiocarcinoma, glioblastoma, hematological neoplasms including acute lymphocytic and myelogenous leukemia, T-cell acute lymphoblastic leukemia/lymphoma, hairy cell leukemia, chronic myelogenous leukemia, multiple myeloma, AIDS-associated leukemias and adult T-cell leukemia/lymphoma, intraepithelial neoplasms including Bowen's disease and Paget's disease, lymphomas including Hodgkin's disease and lymphocytic lymphomas, oral cancer including squamous cell carcinoma, adenoma, angiosarcoma, astrocytoma, epithelial carcinoma, germinoma, glioma, hemangioendothelioma, hemangiosarcoma, hematoma, hepatoblastoma, medulloblastoma, melanoma, parotid gland cancer, neuroblastoma, hepatobiliary cancer, suprarenal cancer, osteosarcoma, retinoblastoma, rhabdomyosarcoma, and teratoma. Furthermore, this term includes acrolentiginous melanoma, actinic keratosis adenocarcinoma, adenoid cystic carcinoma, adenomas, adenosarcoma, adenosquamous carcinoma, astrocytic tumors, Bartholin gland carcinoma, basal cell carcinoma, bronchial gland carcinoma, capillary carcinoid, carcinoma, carcinosarcoma, cystadenoma, endodermal sinus tumor, endometrial hyperplasia, endometrial stromal sarcoma, endometrioid adenocarcinoma, ependymal sarcoma, Ewing sarcoma, focal nodular hyperplasia, germ cell tumors, glucagonoma, hemangioblastoma, hemangioendothelioma, hemangioma, hepatic adenoma, hepatic adenomatosis, hepatocellular carcinoma, insulinoma, intraepithelial neoplasia, squamous cell intraepithelial neoplasia, invasive squamous-cell carcinoma, large cell carcinoma, leiomyosarcoma, malignant melanoma, malignant mesothelial tumor, medulobastoma, medulloepithelioma, mucoepidermoid carcinoma, neuroblastoma, neuroepithelial adenocarcinoma, nodular melanoma, papillary serous adenocarcinoma, pituitary tumors, plasmacytoma, pseudosarcoma, pulmonary blastoma, renal cell carcinoma, retinoblastoma, rhabdomyosarcoma, serous carcinoma, microcytic carcinoma, soft tissue carcinoma, somatostatin secreting tumor, squamous carcinoma, squamous cell carcinoma, undifferentiated carcinoma, uveal melanoma, verrucous carcinoma, vipoma, Wilm tumor, intracerebral cancer, rectal cancer, astrocytoma, microcytic cancer and non-microcytic cancer, metastatic melanoma, androgen-independent metastatic prostate cancer, androgen-dependent metastatic prostate cancer.

Cancer includes, in another embodiment, without limitation, mesothelioma, hepatobiliary (hepatic and biliary duct), bone cancer, pancreatic cancer, skin cancer, cancer of the head or neck, cutaneous or intraocular melanoma, ovarian cancer, colon cancer, rectal cancer, cancer of the anal region, stomach cancer, gastrointestinal (gastric, colorectal, and duodenal), uterine cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, testicular cancer, chronic or acute leukemia, chronic myeloid leukemia, lymphocytic lymphomas, cancer of the bladder, cancer of the kidney or ureter, renal cell carcinoma, carcinoma of the renal pelvis, non-Hodgkins's lymphoma, spinal axis tumors, brain stem glioma, pituitary adenoma, adrenocortical cancer, gall bladder cancer, multiple myeloma, cholangiocarcinoma, fibrosarcoma, neuroblastoma, retinoblastoma, or a combination of one or more of the foregoing cancers.

The term cancer includes both localized tumors as well as metastatic tumors. In an embodiment the cancer is a primary tumor. The term "localized tumor", as used herein, refers to a tumor that originated in the location or organ in which it is present and did not metastasize to that location from another location. Therefore, in an embodiment the cancer to be treated is a non-metastatic cancer. In another embodiment the cancer is a metastatic cancer or cancer metastasis. In the context of the present invention, "metastasis" is understood as the propagation of a cancer from the organ where it stated to a different organ.

In a particular embodiment, the cancer is selected from the group consisting of: breast cancer, prostate cancer, endometrial cancer, ovarian cancer, brain cancer, skin cancer, thyroid cancer, lung cancer, mesothelioma cancer, bladder cancer, pancreatic cancer, colorectal cancer, liver cancer, melanoma, glioblastoma, leukemia and lymphoma.

In a more particular embodiment, the cancer is a lung cancer. Lung cancer is a malignant tumor that begins in the lung. Lung cancer is caused by genetic damage to the DNA of cells in the airways, often caused by cigarette smoking or inhaling damaging chemicals. Damaged airway cells gain the ability to multiply unchecked, causing the growth of a tumor. Eventually lung tumors metastasize, spreading to other parts of the body. Lung cancer is classified based on the type of cells the tumor is derived from; tumors derived from different cells progress and respond to treatment differently. There are two main types of lung cancer, categorized by the size and appearance of the malignant cells seen by a histopathologist under a microscope: small cell lung cancer (SCLC; 15% of cases) and non-small-cell lung cancer (NSCLC; 85% of cases). SCLC tumors are often found near the center of the lungs, in the major airways. Non-small-cell lung cancer (NSCLC), or non-small-cell lung carcinoma, is any type of epithelial lung cancer other than small-cell lung cancer (SCLC). As a class, NSCLCs are relatively insensitive to chemotherapy, compared to small-cell carcinoma.

In a particular embodiment, the lung cancer is a SCLC or a NSCLC. In a more particular embodiment, the lung cancer is a non-small cell lung cancer (NSCLC) or a lung adenocarcinoma.

Adenocarcinoma of the lung or lung adenocarcinoma is currently the most common type of lung cancer in "never smokers" (lifelong nonsmokers). Adenocarcinoma was more often seen peripherally in the lungs than SCLC and squamous-cell lung cancer, both of which tended to be more often centrally located.

In a preferred embodiment, the lung cancer is a lung cancer with at least a mutation in the Epidermal Growth Factor Receptor (EGFR) gene causing resistance to tyrosine kinase inhibitors.

The epidermal growth factor receptor (EGFR; ErbB-1; HER1 in humans) is a transmembrane protein that is a receptor for members of the epidermal growth factor family (EGF family) of extracellular protein ligands. The epidermal growth factor receptor is a member of the ErbB family of receptors, a subfamily of four closely related receptor tyrosine kinases: EGFR (ErbB-1), HER2/neu (ErbB-2), Her 3 (ErbB-3) and Her 4 (ErbB-4). In many cancer types, mutations affecting EGFR expression or activity could result in cancer. Mutations that lead to EGFR constitutive activation have been associated with a number of cancers, including adenocarcinoma of the lung (40% of cases), anal cancers, glioblastoma (50%) and epithelian tumors of the head and neck (80-100%).

In the present invention, the term "resistance to tyrosine kinase inhibitors" refers to the reduction in effectiveness of a tyrosine kinase inhibitor in treating a disease or condition, in particular, in treating cancer.

The term "mutation" as used herein refers to an alteration in the nucleic acid sequence of a gene In a particular embodiment, the lung cancer is a lung cancer with at least one mutation in the EGFR gene (NCBI Gene ID: 1956, 2 July 2023). In another particular embodiment, the lung cancer is a lung cancer with at least two mutation in EGFR. In a particular embodiment, the mutation in the EGFR is a point mutation or small indel. These kind of mutations affect a gene in one or a few nucleotides.

In some embodiments, the mutation is a deletion and/or a substitution.

In a preferred embodiment, the mutation in EGFR is an indel in the exon 19 and/or a L858R point mutation in exon 21.

In a more particular embodiment, the mutation in EGFR is a deletion in the exon 19. Exon 19 deletion (19del) is a sensitive mutation of epidermal growth factor receptor (EGFR) observed in non-small cell lung cancer (NSCLC), and consists of a large number of variants. Ex19del represents in-frame deletions contained amino acid residues 746-750 of the expressed protein (Uniprot accesion number P00533, entry version 287, sequence version 2, 28 June 2023).

In another more particular embodiment, the mutation in EGFR is a L858R mutation in exon 21. L858R mutation is a single-nucleotide substitution that replaced leucine with arginine at codon 858.

In another particular embodiment, the lung cancer is a lung cancer with a deletion in the exon 19 and a L858R mutation in exon 21 of EGFR.

In the present invention, the term "therapy" refers to an anti-cancer treatment comprising the exposure of the patient being treated with an anti-cancer agent, wherein the goal is to prevent or reduce an unwanted physiological change or disease, such as cancer. Beneficial or desired clinical results include, but not limiting, release of symptoms, reduction of the length of the disease, stabilized pathological state (specifically not deteriorated), retardation in the disease's progression, improve of the pathological state and remission (both partial and total), both detectable and not detectable. "Therapy" can mean also to prolong survival, compared to the expected survival if the therapy is not applied. Those who need the therapy include those who are suffering from cancer.

The term "TTKi therapy", in the context of the first method of the invention, refers to any therapy comprising the exposure of the patient being treated with a dual specificity protein kinase TTK inhibitor.

Dual specificity protein kinase TKK (or TKK) also known as Monopolar Spindle 1 Kinase (MPS-1), is a dual specific kinase (tyrosine and serine/threonine) that plays an important role in the cell division cycle by regulating spindle assembly checkpoint during mitosis, which has emerged as a potential target in cancer therapy. In a particular embodiment, the dual specificity protein kinase TKK comprises or consists of the sequence identified by the accession number P33981 (Entry version 223, sequence version 2, 28 June 2023).

The term "dual specificity protein kinase TTK inhibitor" or "TTKi" refers to any molecule capable of completely or partially inhibiting the dual specificity protein kinase TTK expression, both by preventing the expression product of said gene from being produced (interrupting the gene transcription, blocking the translation of the mRNA coming from the gene expression and/or promoting the premature degradation of the mRNA originating from the gene), by directly inhibiting the TTK protein activity and by promoting/targeting the TTK protein degradation by targeting it to the ubiquitin-proteasome pathway or to lysosomal proteolysis.

Suitable methods for determining whether an inhibitor specific for the dual specificity protein kinase TTK is capable of decreasing TTK mRNA levels include, without limitation, standard assays for determining mRNA expression levels such as qPCR, RT-PCR, RNA protection analysis, Northern blot, RNA dot blot, in situ hybridization, microarray technology, tag based methods such as serial analysis of gene expression (SAGE), including variants such as LongSAGE and SuperSAGE, microarrays.

Suitable methods for determining whether an inhibitor acts by decreasing the TTK protein levels include the quantification by means of conventional methods, for example, using antibodies with a capacity to specifically bind to the protein encoded by TTK gene (or to fragments thereof containing antigenic determinants) and subsequent quantification of the resulting antibody-antigen complexes. There are a wide variety of well-known assays that can be used in the present invention, which use non-labeled antibodies (primary antibody) and labeled antibodies (secondary antibodies); among these techniques are included Western blot or Western transfer, ELISA (enzyme linked immunosorbent assay), RIA (radioimmunoassay), competitive EIA (enzymatic immunoassay), DAS-ELISA (double antibody sandwich ELISA), immunocytochemical and immunohistochemical techniques, techniques based on the use of biochips or protein microarrays including specific antibodies or assays based on colloidal precipitation in formats such as dipsticks. Other ways of detecting and quantifying the levels of the protein of interest include techniques of affinity chromatography, binding-ligand assays, etc.

In a particular embodiment, the TTKi is selected from the group consisting of: CFI-402257, AZ-3146, BAY1217389, BAY1161909, NMS-P715, TC Mps1 12, reversine, Mpsl-IN-1, Mpsl-IN-2, Mpsl-IN-3, Mps BAY1, Mps BAY2a, Mps BAY2b, MPI-0479605, S81694/NMS-P153; BOS172722; NTRC 0060-0 and NTRC 1501.

In a more particular embodiment, the TTKi is BAY1217389, BOS172722 or CFI-402257. BAY1217389 is a potent, and selective inhibitor of the monopolar spindle 1 (MPS1) kinase with an IC50 value less than 10 nM. BOS172722 (CCT289346) is a highly potent, selective, and orally bioavailable inhibitor of spindle assembly checkpoint kinase MPS1 (IC50 value of 2 nm). CFI-402257 is a potent (Mps1 Ki = 0.09 ± 0.02 nM; cellular Mps1 EC50 = 6.5 ± 0.5 nM), highly selective, and orally active small-molecule inhibitor of Mps1.

In a first step, the first method of the invention comprises determining the expression level of BH3-interacting domain death agonist (BID) in a sample from a patient suffering from cancer.

The term "sample", as used herein, relates to any sample which can be obtained from the patient and which contains any biological material suitable for detecting DNA, RNA or protein levels. In a particular embodiment, the sample contains genetic material, e.g., DNA, genomic DNA (gDNA), complementary DNA (cDNA), RNA, heterogeneous nuclear RNA (hnRNA), mRNA, etc., from the patient under study. In another particular embodiment the sample contains proteins. The sample can comprise cell and/or non-cell material of the patient. The present method can be applied to any kind of biological sample from a patient, such as a biopsy sample, tissue, cell or biological fluid or biofluid (blood, plasma, serum, saliva, urine, semen, sputum, cerebral spinal fluid (CSF), tears, mucus, sweat, milk), feces, brain extracts, bone marrow, nipple aspirate, samples obtained by bronchial lavage, bronchoscopy, fine needle aspiration biopsy (FNAB), solid tumor biopsy sample, a buccal or buccal pharyngeal swab and the like. Said sample can be obtained by conventional methods, e.g., biopsy, surgical excision or aspiration, by using methods well known to those of ordinary skill in the related medical arts. Methods for obtaining the sample from the biopsy include gross apportioning of a mass, or microdissection or other art-known cell-separation methods. Tumor cells can additionally be obtained from fine needle aspiration cytology. In order to simplify conservation and handling of the samples, these can be formalin-fixed and paraffin-embedded or first frozen and then embedded in a cryosolidifiable medium, such as OCT-Compound, through immersion in a highly cryogenic medium that allows rapid freeze. The samples can also be a cell suspension, cell pellet, cell slide, frozen solid tumor biopsy.

In a particular embodiment, the sample is a tissue sample, preferably a biopsy of a tumor tissue sample, either fresh or frozen.

In a particular embodiment, said sample comprises cancer cells, preferably breast cancer, ovarian cancer, prostate cancer, gastric cancer, pancreatic cancer, lung cancer, colorectal cancer, stomach/gastric cancer, endometrial/uterine/cervical cancer, bladder cancer, head and neck cancer, leukemia, sarcoma, cholangiocarcinoma, glioblastoma, multiple myeloma, lymphoma cells. In a preferred embodiment, the sample comprises lung cancer cells. In a preferred embodiment it is a tumor tissue sample or portion thereof. Preferably, said tumor tissue sample is a breast tumor, ovarian tumor, prostate tumor, gastric tumor, pancreatic tumor, lung tumor, colorectal tumor, stomach/gastric tumor, endometrial/uterine/cervical tumor, bladder tumor, head and neck tumor, sarcoma tumor, cholangiocarcinoma tumor, glioblastoma tumor, multiple myeloma tumor, lymphoma tumor tissue, or portion thereof, sample. In a preferred embodiment, the sample is a lung tumor sample.

In another particular embodiment, the sample from the patient according to the first method of the invention is a biofluid, preferably a biofluid from affected organs.

A "biofluid", "biological fluid sample" or "bodily fluid sample", as used herewith, refers to any biological secretion or fluid, whether physiological or pathological, which is produced in the body of a subject. Such biofluids include, without limitation, blood, plasma, serum, bronchoalveolar washing fluid, urine, nasal secretion, ear secretion, urethral secretion, cerebrospinal fluid, pleural fluid, synovial fluid, peritoneal fluid, ascites fluid, pericardial liquid, amniotic fluid, gastric juice, lymphatic fluid, interstitial fluid, saliva, sputum, liquid deposition, tears, mucus, sweat, milk, semen, vaginal secretions, fluid coming from ulcer, blisters, abscesses and other surface eruptions. Said samples can be obtained by conventional methods, using processes known in the state of art by the person skilled in the art, such as blood extraction, instillation and aspiration of liquid during bronchofibroscopy, cisternal, ventricular or lumbar puncture, pleural puncture or thoracocentesis, joint or synovial percutaneous puncture, abdominal puncture, amniocentesis, expectoration, peritoneal percutaneous puncture, pericardial percutaneous puncture, etc., or by simple harvesting.

In a preferred embodiment, the biofluid is selected from the group consisting of blood, plasma and serum.

The blood sample is typically extracted by means of puncturing an artery or vein, normally a vein from the inner part of the elbow or from the back of the hand, the blood sample being collected in an air-tight vial or syringe. A capillary puncture normally on the heel or on the distal phalanxes of fingers can be performed for analysis by means of a micromethod. Serum can be obtained from the complete blood sample and in the absence of anticoagulant by leaving the sample to settle for 10 minutes so that it coagulates and subsequently centrifuging it at 1,500 rpm for 10 minutes for the purpose of separating the cells (precipitate) from the serum (supernatant). In turn, to obtain the plasma sample the complete blood is contacted with an anticoagulant and is centrifuged at 3,000 rpm for 20 minutes. The precipitate of said centrifugation corresponds to the formed elements, and the supernatant corresponds to the plasma. The serum or the plasma obtained can be transferred to a storage tube for sample analysis.

The term "biomarker" refers to a gene product from a gene of interest, understood as the transcriptional product or the translational product of a gene of interest (i.e. the mRNA transcribed from said gene or the protein encoded by said gene), the amount of which reflects a specific situation, for example, a condition such as the prediction of the response of a patient suffering from cancer to a TTKi therapy. In the present invention the term "biomarker" or "marker" refers to BID.

BH3-interacting domain death agonist (BID) is a member of the Bcl-2 protein family which regulates outer mitochondrial membrane permeability. BID is a pro-apoptotic member that causes cytochrome c to be released from the mitochondria intermembrane space into the cytosol. BID is a pro-apoptotic Bcl-2 protein containing only the BH3 domain. Bcl-2 family members share one or more of the four characteristic domains of homology entitled the Bcl-2 homology (BH) domains (named BH1, BH2, BH3 and BH4), and can form hetero- or homodimers. Bcl-2 proteins act as anti- or pro-apoptotic regulators that are involved in a wide variety of cellular activities.

As it is used herein, the term "expression level" refers to the expression level of a gene product, more specifically to a measurable quantity of a gene product produced by a specific gene in a specific sample of the patient. The term "gene product", as used herein, refers to a transcriptional product or a translational product, and thus corresponds to the mRNA transcribed from said gene or to the protein encoded by a specific gene.

As the person skilled in the art understands, the expression levels of BID can be measured by determining the mRNA expression levels of the gene or by determining the protein levels encoded by said gene. In a particular embodiment, said value can be determined by measuring the mRNA level of the gene of interest or a fragment thereof or by measuring the amount of protein encoded by said gene of interest or a variant thereof.

In a particular embodiment, the expression level of BID is determined by measuring the BID mRNA levels or of a fragment thereof.

In order to measure the mRNA levels of BID, the biological sample may be treated to physically, mechanically or chemically disrupt tissue or cell structures, to release intracellular components into an aqueous or organic solution to prepare nucleic acids for further analysis. The nucleic acids are extracted from the sample by procedures known to the skilled person using commercially available reagents. RNA is then extracted from frozen or fresh samples by any of the methods typical in the art, for example, Sambrook, J., et al., 2001. Molecular cloning: A Laboratory Manual, 3rd ed., Cold Spring Harbor Laboratory Press, N.Y., Vol. 1-3. Preferably, care is taken to avoid degradation of the RNA during the extraction process.

The expression level can be determined using mRNA obtained from a formalin-fixed, paraffin-embedded tissue sample. mRNA may be isolated from an archival pathological sample or biopsy sample which is first deparaffinized. An exemplary deparaffinization method involves washing the paraffinized sample with an organic solvent, such as xylene. Deparaffinized samples can be rehydrated with an aqueous solution of a lower alcohol. Suitable lower alcohols, for example, include methanol, ethanol, propanols and butanols. Deparaffinized samples may be rehydrated with successive washes with lower alcoholic solutions of decreasing concentration, for example. Alternatively, the sample is simultaneously deparaffinized and rehydrated. The sample is then lysed and RNA is extracted from the sample. Samples can be also obtained from fresh tumor tissue such as a resected tumor. In a particular embodiment samples can be obtained from fresh tumor tissue or from OCT embedded frozen tissue.

Suitable methods to determine gene expression levels at the mRNA level include, without limitation, standard assays for determining mRNA expression levels such as qPCR, RT-PCR, RNA protection analysis, Northern blot, RNA dot blot, TaqMan^{®}, tag based methods such as serial analysis of gene expression (SAGE) including variants such as LongSAGE and SuperSAGE, microarrays, nucleic acid sequence based amplification (NASBA).

In a particular embodiment, the determination of the expression level of BID is carried out by RT-qPCR or nCounter.

In another particular embodiment, the expression level of BID is determined by measuring the expression level of the protein encoded by this gene, because the increased expression of a gene is usually accompanied by an increase in the amount of corresponding protein. The determination of the amount of a protein corresponding to the expression of a specific gene can be performed using any conventional method for protein detection and quantification, for example by means of an immunoassay, etc. By way of non-limiting illustration, said determination can be performed using antibodies with the capability to bind specifically to the protein to be determined (or fragments thereof with the antigenic determinants) and subsequent quantification of the antigen-antibody complex derivatives. The antibodies can be, for example, polyclonal sera, hybridoma supernatants or monoclonal antibodies, fragments of antibodies, Fv, Fab, Fab' and F(ab')2, scFv, diabodies, triabodies, tetrabodies, humanized antibodies, etc. Said antibodies may (or may not) be labeled with a marker. Illustrative, non-limiting examples of markers that can be used in the present invention include radioactive isotopes, enzymes, fluorophores, chemiluminescent reagents, enzyme cofactors, enzyme substrates, enzyme inhibitors, etc. There is a wide range of well-known assays that can be used in the present invention, such as, for example, assays based on Western-blot or immunoblot techniques, ELISA (enzyme-linked immunosorbent assay), RIA (radioimmunoassay), EIA (enzyme immunoassay), DAS-ELISA (double antibody sandwich ELISA), immunocytochemical or immunohistochemical techniques, etc. Other ways of detecting and quantifying the protein include affinity chromatogaphy, ligand binding assay techniques, particle-enhanced turbidimetric immunoassay (PETIA), etc.

In a particular embodiment, the determination of the expression level of BID is carried out by immunohistochemical techniques.

The second step of the first method of the invention comprises comparing the expression level of BID obtained in the first step of said method with a reference value.

The term "reference value", as used herein, refers to a laboratory value used as a reference for values/data obtained by means of samples collected from subjects. The reference value or reference level can be an absolute value, a relative value, a value that has an upper or a lower limit, a range of values, an average value, a median value, a mean value, or a value as compared to a particular control or baseline value. A reference value can be based on an individual sample value, such as, for example, a value obtained from a sample from the subject being tested, but at an earlier point in time. The reference value can be based on a large number of samples, for example on a group of patients considered to be representative, such as the values obtained from a population of subjects of matched group coinciding with that of the patient object of the study, or based on a pool of samples including or excluding the sample to be tested.

In the context of the method of the invention for the prediction of response of a patient suffering from cancer to a TTKi therapy, a preferred reference value can be the BID expression level determined in a tumor sample from a patient having cancer or having had cancer which has shown a good response to treatment with the TTKi therapy, said expression levels having been determined at the time that the patient was being treated. In another embodiment, the reference value can be the BID expression level determined in a tumor sample from a patient having cancer or having had cancer which has shown a poor response to treatment with the TTKi therapy, said expression levels having been determined at the time that the patient was being treated. In another embodiment, the reference value can be the BID expression level determined in a tumor sample from a pool of patients having cancer or having had cancer wherein several of them have shown good response and the others have shown bad response to treatment with the TTKi therapy, said expression levels having been determined right before the patient was being treated. In another embodiment, the reference value can be the BID expression level in a healthy patient, i.e., a patient which has not been diagnosed with the type of cancer for which a prediction of the response to therapy is desired.

Once this reference value is established, the level of the biomarker, i.e. BID, expressed in the sample can be compared with said reference value, and thus be assigned a level of "increased", "decreased" or "equal" expression level.

In the context of the present invention, it is considered that an expression level of BID in the sample from the patient is "above", "increased" or "greater than" the reference value for said marker when the expression level of this gene in the sample from the patient increases, for example, 5%, 10%, 25%, 50%, 100% or even more when compared with the reference value for said gene, or when it increases, for example, at least 1.1-fold, 1.5-fold, 5-fold, 10-fold, 20-fold, 30-fold, 40-fold, 50-fold, 60-fold, 70-fold, 80-fold, 90-fold, 100-fold or even more compared with the reference value of this marker.

When a comparison between the expression level of BID in the sample from the patient and the reference value for said marker has been made, the first method of the invention allows making a prediction as to whether the patient will show a good response to a TTKi therapy. Thus, patients having an increased level of BID with respect to the reference value are predicted to have a good response to the TTKi therapy.

### In vitro method for designing a personalized therapy for a patient

The authors of the present invention have observed that an increased expression of BID with respect to a reference value is indicative of a good response to a TTKi therapy in cell lines and animal models of human tumors. Therefore, the detection of a high level of said biomarker can be a marker useful for selecting a patient for being treated with a therapy based on a TTKi. In this way, patients can proceed directly to adequate therapies while avoiding less effective therapies or secondary effects associated to them.

Thus, in a second aspect, the present invention relates to an *in vitro* method, hereinafter the second method of the invention, for designing a personalized therapy for a patient suffering cancer that comprises:
i) determining the expression level of BH3-interacting domain death agonist (BID) in a sample from said patient and,
ii) comparing the expression level obtained in (i) to a reference value,
wherein an expression level of BID above a reference value is indicative that the patient is to be treated with a TTKi.

The term "designing a personalized therapy" or "selecting a personalized therapy", as used herein, refers to selecting or designing appropriate and optimal therapies based on a patient's specific medical characteristics, resulting from its personal background. In the context of the present invention, said characteristic consists of the level of BID in a sample isolated from the patient.

The terms *"in vitro"* and "patient" or "subject" have been defined or explained in the first method of the invention, and these definitions are applicable to the second method of the invention.

The patients tested in the second method of the invention have been previously diagnosed with cancer. The term "cancer" has been defined or explained in the first method of the invention and this definition is applicable to the second method of the invention.

In a particular embodiment, the cancer is selected from the group consisting of: breast cancer, prostate cancer, endometrial cancer, ovarian cancer, brain cancer, skin cancer, thyroid cancer, lung cancer, mesothelioma cancer, bladder cancer, pancreatic cancer, colorectal cancer, liver cancer, melanoma, glioblastoma, leukemia and lymphoma.

In a more particular embodiment, the cancer is a lung cancer. The term "lung cancer" has been defined or explained in the first method of the invention and this definition is applicable to the second method of the invention. In a particular embodiment, the lung cancer is a SCLC or a NSCLC. In a more particular embodiment, the lung cancer is a non-small cell lung cancer (NSCLC) or a lung adenocarcinoma.

In a preferred embodiment, the lung cancer is a lung cancer with at least a mutation in the Epidermal Growth Factor Receptor (EGFR) gene causing resistance to tyrosine kinase inhibitors.

The terms "EGFR", "mutation" and "resistance" have been defined or explained in the first method of the invention, and these definition are applicable to the second method of the invention. In a preferred embodiment, the mutation in EGFR is a deletion in the exon 19 and/or a L858R mutation in exon 21.

In a more particular embodiment, the mutation in EGFR is a deletion in the exon 19.

In another more particular embodiment, the mutation in EGFR is a L858R mutation in exon 21.

In another particular embodiment, the lung cancer is a lung cancer with a deletion in the exon 19 and a L858R mutation in exon 21 of EGFR.

The term "TTKi" and "TTKi therapy" have been defined or explained in the first method of the inventions, and these definitions are applicable to the second method of the invention.

In a particular embodiment, the TTKi is selected from the group consisting of: CFI-402257, AZ-3146, BAY1217389, BAY1161909, NMS-P715, TC Mps1 12, reversine, Mpsl-IN-1, Mpsl-IN-2, Mpsl-IN-3, Mps BAY1, Mps BAY2a, Mps BAY2b, MPI-0479605, S81694/NMS-P153; BOS172722; NTRC 0060-0 and NTRC 1501. In a more particular embodiment, the TTKi is BAY1217389, BOS172722 or CFI-402257.

The first step of the second method of the invention comprises determining the level of BID in a sample from said patient; and the second step comprises comparing the level of said biomarker with a reference value.

The terms "sample", "BID", "expression level", "biomarker" "reference value" have been defined or explained in the first method of the invention, and these definitions are applicable to the second method of the invention.

In a particular embodiment, the sample is a tissue sample, preferably a biopsy of a tumor tissue sample, either fresh or frozen. In a preferred embodiment, the sample is a lung tumor sample.

In another particular embodiment, the sample from the patient according to the second method of the invention is a biofluid, preferably a biofluid from affected organs. In a preferred embodiment, the biofluid is selected from the group consisting of blood, plasma and serum.

In a particular embodiment, the expression level of BID is determined by measuring the BID mRNA levels or of a fragment thereof. In a more particular embodiment, the determination of the expression level of BID is carried out by RT-qPCR or nCounter.

In another particular embodiment, the expression level of BID is determined by means of determining the expression level of the protein encoded by this gene. In a more particular embodiment, the determination of the expression level of BID is carried out by immunohistochemical techniques.

When a comparison between the level of BID and the reference value has been made, the second method of the invention allows deciding the suitable treatment for the patient tested. Thus, patients having an increased level of BID with respect to the reference value should select a therapy comprising a TTKi.

On the other hand, patients having an equal or a decreased level of BID with respect to the reference value should select an alternative therapy, i.e., a therapy that does not comprise a TTKi.

The expression "alternative therapy", in the context of the second method of the invention, refers to any therapy different from a therapy comprising a TTKi. Alternative therapy, includes, without limitation, surgery, chemotherapy, radiation therapy, hormonal therapy and targeted therapy, including immunotherapy, and combinations thereof.

Illustrative, non-limitative examples of alternative therapies include, surgery, treatments with anti-cancer agents such as chemotherapy agents including anthracycline antibiotics such as doxorubicin and daunorubicin, taxanes such as Taxol^{™} and docetaxel, vinca alkaloids such as vincristine and vinblastine, 5-fluorouracil (5-FU), leucovorin, irinotecan, idarubicin, mitomycin C, oxaliplatin, raltitrexed, tamoxifen, cisplatin, carboplatin, methotrexate, actinomycin D, mitoxantrone, blenoxane or mithramycin; toxins such as the ricin A-chain, saponin, the diphtheria A-chain, active non-binding fragments of the diphtheria toxin, Pseudomonas aeruginosa exotoxin A-chain, abrin A-chain, modecin A-chain, α-sarcin, Leurites fordii A-proteins, dianthin proteins, Phytolaca americana (PAPI, PAPII and PAP-S) proteins, Momordica charantia inhibitor, curcine, crotin, Saponaria officinalis inhibitor, gelonin, mitogelin, restrictocin, phenomycin, enomycin and trichothecenes; enzymes such as alkaline phosphatase which activates etoposide and doxorubicin; carboxypeptidase G2 which activates nitrogen mustards; beta-lactamase which activates doxorubicin, paclitaxel and mitomycin; cytokines such as TNF factor alpha, INF-gamma, GM-GSF factor or IL-2; radioisotopes such as 1311, 90Y, 177Lu, 188Re, 67Cu, 211At, 213Bi, 1251, 111In; antiangiogenic agents such as paclitaxel, 2-methoxyestradiol, prinomastat, batimastat, carboxyamidotriazole, CC-1088, dextromethorphan acetic acid, dimethylxanthenone acetic acid, endostatin, IM-862, marimastat, penicillamine, PTK787/ZK 222584, RPI.4610, squalamine lactate, SU5416, thalidomide, combretastatin, tamoxifen, COL-3, neovastat, BMS-275291, SU6668, anti-VEGF antibodies, Medi-522 (Vitaxin II), CAI, interleukin 12, IM862, amiloride, angiostatin, KI-3 angiostatin, KI-5 angiostatin, Captopril, DL-alpha-difluoromethylornithine, DL-alpha-difluoromethylornithine HCl, endostatin, fumagillin, herbimycin A, 4-Hydroxyphenylretinamide, juglone, laminin, laminin hexapeptide, laminin pentapeptide, lavendustin A, medroxyprogesterone, minocycline, placenta ribonuclease inhibitor, suramin, thrombospondin, antibodies directed against proangiogenic factors (for example, Avastin, Erbitux, Vectibix, Herceptin); low molecular weight tyrosine kinase inhibitors of proangiogenic growth factors (for example Tarceva, Nexavar, Sutent, Iressa); mTOR inhibitors (for example Torisel); interferon alpha, beta and gamma, IL-12, matrix metalloproteinase inhibitors (for example, COL3, marimastat, batimastat); ZD6474, SUI1248, vitaxin; PDGFR inhibitors (for example Gleevec); NM3 and 2- ME2; cyclopeptides such as cilengitide; antiproliferative agents such as (i) antimetabolites such as folic acid antimetabolites (aminopterin, denopterin, methotrexate, edatrexate, trimetrexate, nolatrexed, lometrexol, pemetrexed, raltitrexed, piritrexim, pteropterin, leucovorin, 10-propargyl-5,8-dideazafolate (PDDF, CB3717)), purine analogs (cladribine, clofarabine, fludarabine, mercaptopurine, pentostatin, thioguanine) and pyrimidine analogs (capecitabine, cytarabine or ara-C, decitabine, fluorouracil, 5-fluorouracil, doxifluridine, floxuridine and gemcitabine) (ii) natural products, such as antitumor antibiotics and mitotic inhibitors such vinca alkaloids such as vindesine, vincristine, vinblastine, vinorelbine; taxanes such as paclitaxel (Taxol^{™}), docetaxel (Taxotere^{™}); colchicine (NSC 757), thiocolchicine (NSC 361792), colchicine derivatives (e. g., NSC 33410), and allocolchicine (NSC 406042); halichondrin B (NSC 609395); dolastatin 10 (NSC 376128); maytansine (NSC 153858); rhizoxin (NSC 332598); epothilone A, epothilone B; discodermolide; estramustine; nocodazole; (iii) hormones and antagonist thereof, such tamoxifen, toremifene, anastrozole, arzoxifene, lasofoxifene, raloxifene, nafoxidine, fulvestrant, aminoglutethimide, testolactone, atamestane, exemestane, fadrozole, formestane, letrozole, goserelin, leuprorelin or leuprolide, buserelin, histrelin, megestrol and fluoxymesterone; (iv) biological agents, such as viral vectors, interferon alpha and interleukines; (v) platinum based compounds such as carboplatin, cisplatin [cis-diamminedichloroplatinum, (CDDP)], oxaliplatin, iproplatin, nedaplatin, triplatin tetranitrate, tetraplatin, satraplatin (JM216), JM118 [cis ammine dichloro (II)], JM149 [cis ammine dichloro (cyclohexylamine) trans dihydroxo platinum (IV)], JM335 [trans ammine dichloro dihydroxo platinum (IV)], transplatin, ZD0473, cis, trans, cis-Pt(NH3)(C6H11NH2)(OOCC3H7)2Cl, malanate-I,2-diaminociclohexanoplatin(II), 5-sulphosalycilate-trans-(I,2- diaminociclohexane)platin (II) (SSP), poly-[(trans-I,2-diaminocyclohexane)platin]- carboxyamilose (POLY-PLAT) and 4-hydroxy-sulphonylphenylacetate (trans- 1,2- diaminocyclohexane) platinum (II) (SAP) and the like and (vi) DNA-alkylating drugs such as nitrogen mustards, nitrosoureas, ethylenimine derivatives, alkyl sulfonates and triazenes, including, but not limited to, cyclophosphamide (Cytoxan^{™}), busulfan, improsulfan, piposulfan, pipobroman, melphalan (L-sarcolysin), chlorambucil, mechlorethamine or mustine, uramustine or uracil mustard, novembichin, phenesterine, trofosfamide, ifosfamide, carmustine (BCNU), lomustine (CCNU), chlorozotocin, fotemustine, nimustine, ranimnustine, semustine (methyl-CCNU), streptozocin, thiotepa, triethylenemelamine, triethylenethiophosphoramine, procarbazine, altretamine, dacarbazine, mitozolomide and temozolomide; targeted therapy or immunotherapy such as anti-PD1 or anti-CTLA4.

### Pharmaceutical compositions and uses thereof for the treatment of cancer

The authors of the present invention have found that a combination of a TTKi and a BH3 mimetic agent results in a synergistic effect in the antitumor activity. Accordingly, in another aspect, the present invention relates to a pharmaceutical composition, hereinafter the pharmaceutical composition of the invention, comprising a TTKi, a BH3 mimetic agent and at least one pharmaceutically acceptable excipient.

The term "TTKi" has been defined or explained in the first method of the invention and this definition is applicable to the pharmaceutical composition of the invention.

In a particular embodiment, the TTKi is selected from the group consisting of: CFI-402257, AZ-3146, BAY1217389, BAY1161909, NMS-P715, TC Mps1 12, neversine, Mps1-IN-1, Mps1-IN-2, Mps1-IN-3, Mps BAY1, Mps BAY2a, Mps BAY2b, MPI-0479605, SP600125, S81694/NMS-P153; BOS172722; NTRC 0060-0 and NTRC 1501.

In a more particular embodiment, the TTKi is BAY1217389, BOS172722 or CFI-402257.

The term "BH3 mimetic", as used herein, include molecules which mimic the chemical structure of the BH3 structure and retain the functional properties of the BH3 structure. BH3 mimetics mimic the BH3-only proteins by inserting their BH3 domain into the hydrophobic groove of the prosurvival BCL-2 proteins, inhibiting their functional activity, and thus inducing apoptosis. Approaches to designing BH3 analogs, derivatives and mimetics, such as natural library screening, peptide therapeutics and structure-based design are known in the art. These different approaches can be used exclusively or in a combined manner.

In a particular embodiment, the BH3 mimetic agent is selected from the group consisting of: ABT-737 (CAS: 852808-04-9), ABT-263 (navitoclax, CAS: 923564-51-6), gossypol (CAS: 90141-22-3), obatoclax (GX15-070, CAS: 803712-67-6), venetoclax (CAS: 1257044-40-8), A1155463 (CAS: 1235034-55-5), A-385358 (CAS: 406228-55-5), BM-957 (CAS: 1391107-54-2), WEHI-539 (CAS: 1431866-33-9) and the BIM BH₃ peptide SAHB_{A}.

In some embodiments, the TTKi and the BH3 mimetic are each in a separate preparation. In some embodiments, the TTKi and the BH3 mimetic are administered simultaneously, sequentially or alternately.

In some embodiments, the combination product is in the form of a pharmaceutical composition. The pharmaceutical composition of the invention, in addition to comprising a TTKi and a BH3 mimetic, comprises a pharmaceutically acceptable excipient.

In some embodiments, the concentration of any one TTKi and BH3 mimetic provided in a pharmaceutical composition of the invention, is independently in the range from about 0.001% to about 10%, from about 0.01% to about 5%, from about 0.02% to about 4.5%, from about 0.03% to about 4%, from about 0.04% to about 3.5%, from about 0.05% to about 3%, from about 0.06% to about 2.5%, from about 0.07% to about 2%, from about 0.08% to about 1.5%, from about 0.09% to about 1%, from about 0.1% to about 0.9% w/w, w/v, or v/v of the pharmaceutical composition.

The expression "pharmaceutically acceptable excipient" or "pharmaceutically acceptable carrier", as used herein, refers to any compound or combination of compounds that is essentially non-toxic to the subject at the dosage and concentration employed, and is compatible with the other components of a medicament. Thus, an excipient is an inactive substance formulated alongside the active ingredient of a medicament for the purpose of bulking-up compositions that contain said active ingredients. Excipients also can serve various therapeutic enhancing purposes, such as facilitating the active ingredient absorption or solubility, or other pharmacokinetic considerations. Excipients can also be useful in the manufacturing process, to aid in the handling of the active substance concerned such as by facilitating powder flowability or non-stick properties, in addition to aiding in vitro stability such as prevention of denaturation over the expected shelf life.

The "dosage form" is the configuration to which the active ingredients and excipients are adapted to provide a pharmaceutical composition or medicinal product. It is defined by the combination of the form in which the pharmaceutical composition is presented by the manufacturer and the form in which it is administered.

The pharmaceutical composition of the invention comprises the TTKi and the BH3 mimetic in a therapeutically effective amount.

The term "therapeutically effective amount", as used herein, refers to the sufficient amount of the agents (TTKi and BH3 mimetic) to provide the desired effect and will generally be determined by, among other causes, the characteristics of the compound itself and the therapeutic effect to be achieved. It will also depend on the subject to be treated, the severity of the cancer disease suffered by said subject, the chosen dosage form, administration route, etc.

In another aspect, the present invention relates to the pharmaceutical composition of the invention for use in the treatment of cancer.

The term "treat" or "treatment" refers to a therapeutic treatment, as well as a prophylactic or prevention method, wherein the goal is to prevent or reduce an unwanted physiological change or disease, such as cancer. Beneficial or desired clinical results include, but not limiting, release of symptoms, reduction of the length of the disease, stabilized pathological state (specifically not deteriorated), retardation in the disease's progression, improve of the pathological state and remission (both partial and total), both detectable and not detectable. "Treatment" can mean also to prolong survival, compared to the expected survival if the treatment is not applied. Those who need the treatment include those who are suffering from cancer.

The compositions for use in a method for treating cancer involve the co-administration of the compounds forming part of the composition. The terms "co-administration" or the like, as used herein, are meant to encompass administration of the selected therapeutic agents to a single patient, and are intended to include treatment regimens in which the agents are administered by the same or different route of administration or at the same or different time.

The term "cancer" has been defined or explained in the first method of the invention and this definition is applicable to the present aspect.

In a particular embodiment, the cancer is selected from the group consisting of: breast cancer, prostate cancer, endometrial cancer, ovarian cancer, brain cancer, skin cancer, thyroid cancer, lung cancer, mesothelioma cancer, bladder cancer, pancreatic cancer, colorectal cancer, liver cancer, melanoma, glioblastoma, leukemia and lymphoma.

In a more particular embodiment, the cancer is a lung cancer. In a particular embodiment, the lung cancer is a SCLC or a NSCLC. In a still more particular embodiment, the lung cancer is a non-small cell lung cancer or a lung adenocarcinoma.

In a preferred embodiment, the lung cancer is a lung cancer with at least a mutation in the Epidermal Growth Factor Receptor (*EGFR*) gene causing resistance to tyrosine kinase inhibitors. In a more preferred embodiment, the mutation in the *EGFR* gene is a deletion in the exon 19 or a L858R mutation in exon 21.

In a more particular embodiment, the mutation in EGFR is a deletion in the exon 19.

In another more particular embodiment, the mutation in EGFR is a L858R mutation in exon 21.

In another particular embodiment, the lung cancer is a lung cancer with a deletion in the exon 19 and a L858R mutation in exon 21 of EGFR.

The terms "mutation" and resistance" have defined or explained in the first method of the invention and these definitions are applicable to the pharmaceutical composition of the invention.

In a particular embodiment, the pharmaceutical composition of the invention is administered intradermally, subcutaneously, via dermal infusion, via subcutaneous infusion, intravenously, or orally.

The pharmaceutical composition may be in a form suitable for oral use, for example, as tablets, troches, lozenges, aqueous or oily suspensions, dispersible powders or granules, emulsions, solutions, hard or soft capsules, or syrups or elixirs. Compositions intended for oral use may be prepared according to any method known to the art for the manufacture of pharmaceutical compositions and such compositions may contain one or more agents selected from the group consisting of sweetening agents, flavoring agents, coloring agents and preserving agents in order to provide pharmaceutically elegant and palatable preparations.

Formulations for oral use may also be presented as hard gelatine capsules wherein the active ingredient is mixed with an inert solid diluent, for example, calcium carbonate, calcium phosphate or kaolin, or as soft gelatine capsules wherein the active ingredient is mixed with water or an oil medium, for example peanut oil, liquid paraffin, or olive oil.

Aqueous suspensions contain the active materials in a mixture with excipients suitable for the manufacture of aqueous suspensions. Such excipients are suspending agents, for example sodium carboxymethylcellulose, methylcellulose, hydroxypropylmethylcellulose, sodium alginate, polyvinyl-pyrrolidone, gum tragacanth and gum acacia; dispersing or wetting agents may be a naturally-occurring phosphatide, for example lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or n-propyl, p-hydroxybenzoate, one or more colouring agents, one or more flavouring agents, and one or more sweetening agents, such as sucrose or saccharin.

Oily suspensions may be formulated by suspending the active ingredient in a vegetable oil, for example arachis oil, olive oil, sesame oil or coconut oil, or in a mineral oil such as liquid paraffin. The oily suspensions may contain a thickening agent, for example beeswax, hard paraffin or cetyl alcohol. Sweetening agents such as those set forth above, and flavouring agents may be added to provide a palatable oral preparation. These compositions may be preserved by the addition of an anti-oxidant such as ascorbic acid.

The pharmaceutical composition may be in the form of a sterile injectable aqueous or oleaginous suspension. This suspension may be formulated according to the known art using those suitable dispersing or wetting agents and suspending agents which have been mentioned above. The sterile injectable preparation may also be a sterile injectable solution or suspension in a non-toxic parenterally-acceptable diluent or solvent, for example as a solution in 1,3-butane diol. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono- or diglycerides. In addition, fatty acids such as oleic acid find use in the preparation of injectables.

Furthermore, various systems are known which can be used for sustained release administration of the pharmaceutical composition, including, without limitation, encapsulation in liposomes, microbubbles, microparticles or microcapsules and the like. Suitable sustained release forms, as well as materials and methods for their preparation, are well known in the prior art. Thus, the orally administrable form of the pharmaceutical composition is in a sustained release form further comprising at least one coating or matrix. The sustained release coating or matrix includes, without limitation, semi-synthetic or synthetic, water insoluble or modified natural polymers, waxes, fats, fatty alcohols, fatty acids, natural, semi-synthetic or synthetic plasticisers or a combination of two or more thereof. Enteric coatings can be applied by conventional processes known to the person skilled in the art.

For topical use, creams, ointments, jellies, solutions or suspensions and the like, containing the TTKi and the BH3 mimetics are employed. Similarly, transdermal patches may also be used for topical administration.

It will be understood, however, that the specific dose level and frequency of dosage for any particular patient may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the host undergoing therapy.

The pharmaceutical composition may be used in combination with one or more other drugs in the treatment of cancer.

Such other compound(s)/drug(s) may be administered by a route and in an amount commonly used therefore, contemporaneously or sequentially with the pharmaceutical composition of the invention. However, the combination therapy may also include therapies in which the pharmaceutical composition and one or more other compounds are administered on different overlapping schedules. It is also contemplated that when used in combination with one or more other active ingredients, the pharmaceutical composition and the other active ingredients may be used in lower doses than when each is used singly. Accordingly, the pharmaceutical compositions include those that contain one or more other active ingredients, in addition to the TTKi and BH3 mimetic.

Thus, in a particular embodiment, the pharmaceutical composition of the invention is administered in combination with at least another suitable compound or agent for the treatment of cancer, wherein said at least another suitable compound or agent is administered simultaneously, sequentially or separately.

Examples of other suitable compounds or agents for the treatment of cancer include, but are not limited to, chemotherapeutic agents, hormonal agents, immune stimulants BCG and levamisole, cytokines, monoclonal antibodies, radiolabeled antibodies, DC vaccines, CART-T cell or NK therapy.

### Uses of the reagents specific for the determination of the expression level of BID

In another aspect, the present invention relates to reagents specific for the determination of the expression level of BID for predicting the response of a patient suffering from cancer to a dual specificity protein kinase TTK inhibitor (TTKi) therapy or for designing a personalized therapy for a patient suffering cancer.

The term "reagent", as used herein, refers to any compound or composition which can be used for detecting the level of any BID. The reagent can include, optionally, reagents for detecting one or more housekeeping genes or the protein encoded by said housekeeping gene(s).

The expression "reagent specific for determining the expression level", as used herein, refers to any compound or set of compounds that allows the specific determination of the expression level of a gene or protein by detection methods well known by the person skilled in the art. Particularly, the term "specific reagent", as used herein, refers to any reagent that can be used for the specific quantification of BID.

In an embodiment, the reagent specific for determining the expression level of BID is a nucleic acid capable of specifically hybridizing with BID gene or mRNA. In a preferred embodiment said nucleic acid is an oligonucleotide that hybridize specifically to BID.

The term "oligonucleotide", as used herein, refers to a nucleic acid having preferably at least 10 nucleotides, preferably at least 15 nucleotides, preferably at least 20 nucleotides, preferably at least 25 nucleotides and preferably no more than 100 nucleotides, including polyribonucleotides, polydeoxyribonucleotides and combinations thereof. The term "oligonucleotide" also refers to molecules formed by conventional nucleotides bound by phosphodiester conventional bonds as well as variants thereof including modifications in purines or pyrimidines or modifications in riboses or deoxyriboses designed to increase the stability of the oligonucleotide. Alternatively or additionally, the oligonucleotides may contain modified bonds such as phosphotriester, phosphorothioate, methylphosphonate, etc., or may be peptide nucleic acids (PNA). Methods to synthesize oligonucleotides are well known by the person skilled in the art. The oligonucleotide can be a primer or a probe. In an embodiment the oligonucleotide is a probe. In another embodiment the oligonucleotide is a primer, preferably a pair of primers. In an embodiment, the present invention provides a set of one or more pairs of oligonucleotide primers designed to specifically amplify the biomarker of the methods of the invention, i.e. BID.

Nucleic acids capable of specifically hybridizing with the BID gene are, for example, one or more pairs of primer oligonucleotides for the specific amplification of fragments of the mRNA (or of their corresponding cDNA) of said gene.

As the skilled person understands, the oligonucleotide primers and probes can be used in all techniques of gene expression profiling (RT-PCR, SAGE, TaqMan, Real Time-PCR, NASBA, etc.).

In another embodiment, the reagent specific for determining the expression level of BID is a compound that specifically binds to the BID protein, particularly is selected from the group consisting of antibodies, aptamers and fragments thereof.

In a preferred embodiment, the reagent specific for determining the expression level of BID protein is an antibody or fragment thereof capable of specifically binding to (i.e., specifically recognizing) BID proteins or to variants thereof (including fragments thereof containing antigenic determinants).

Antibodies, or fragments thereof, capable of detecting an antigen, capable of specifically binding to a protein or to variants thereof are, for example, monoclonal and polyclonal antibodies, antibody fragments, Fv, Fab, Fab' and F(ab')2, ScFv, diabodies, triabodies, tetrabodies and humanised antibodies. The antibodies can be used in conventional methods for detecting protein expression levels, such as Western-blot or Western transfer, ELISA (enzyme linked immunosorbent assay), RIA (radioimmunoassay), competitive EIA (enzymatic immunoassay), DAS-ELISA (double antibody sandwich ELISA), immunocytochemical and immunohistochemical techniques, techniques based on the use of biochips, protein microarrays including specific antibodies or assays based on colloidal precipitation in formats such as dipsticks, etc. In a preferred embodiment said antibodies are used in an immunoassay.

### Method for the personalized therapy of a cancer patient

In another aspect, the present invention relates to a method, hereinafter the third method of the invention, for predicting the response of a patient suffering from cancer to a TTKi therapy and treating the patient that comprises:
a) determining the expression level of BH3-interacting domain death agonist (BID) in a sample from said patient; and
b) comparing the expression level obtained in (i) to a reference value,
wherein if the expression level of BID in the sample from the patient is above a reference value, said patient is treated with a TTKi.

The terms "sample", "patient" or "subject", "cancer", "TTKi", "TTKi therapy" and "BID" have been explained or defined in the first method of the invention, and these definitions are applicable to the third method of the invention.

In a first step, the third method of the invention comprises determining the expression level of BH3-interacting domain death agonist (BID) in a sample from a patient suffering from cancer.

The term "expression level" has been defined or explained in the first method of the invention and this definition is applicable to the third method of the invention. In a particular embodiment, the expression level of BID is determined by measuring the BID mRNA levels or of a fragment thereof. In a more particular embodiment, the determination of the expression level of BID is carried out by RT-qPCR or nCounter.

In another particular embodiment, the expression level of BID is determined by means of determining the expression level of the protein encoded by this gene. In a more particular embodiment, the determination of the expression level of BID is carried out by immunohistochemical techniques.

The second step of the third method of the invention comprises comparing the expression level of BID obtained in the first step of said method with a reference value. The term "reference value" has been defined or explained in the first method of the invention, and this definition is applicable to the third method of the invention.

When a comparison between the expression level of BID in the sample from the patient and the reference value for said marker has been made, the third method of the invention allows making a prediction as to whether the patient will show a good response to a TTKi therapy. Thus, patients having an increased level of BID with respect to the reference value will have a good response to the TTKi therapy, and then a TTKi therapy is administered to said patient who will benefit from the administration of said therapy.

The term "treated" or "treatment" has been defined or explained in the pharmaceutical composition of the invention and this definition is applicable to the third method of the invention.

In further aspects, the present invention relates to:
1. An *in vitro* method for predicting the response of a patient suffering cancer to a dual specificity protein kinase TTK inhibitor (TTKi) therapy that comprises:
   i) determining the expression level of BH3-interacting domain death agonist (BID) in a sample from said patient and,
   ii) comparing the expression level obtained in (i) to a reference value,
   wherein if the expression level of BID in a sample from the patient is above a reference value, it indicates that the patient is predicted to respond to the TTKi therapy.
2. An *in vitro* method for designing a personalized therapy for a patient suffering cancer that comprises:
   i) determining the expression level of BH3-interacting domain death agonist (BID) in a sample from said patient and,
   ii) comparing the expression level obtained in (i) to a reference value,
   wherein an increased expression level of BID above a reference value is indicative that the patient is to be treated with a TTKi.
3. The method according to any one of aspects 1 or 2, wherein the TTKi is selected from the group consisting of: CFI-402257, AZ-3146, BAY1217389, BAY1161909, NMS-P715, TC Mps1 12, reversine, Mpsl-IN-1, Mpsl-IN-2, Mpsl-IN-3, Mps BAY1, Mps BAY2a, Mps BAY2b, MPI-0479605, S81694/NMS-P153; BOS172722; NTRC 0060-0 and NTRC 1501.
4. The method according to aspect 3, wherein the TTKi is BAY1217389, BOS172722 or CFI-402257.
5. The method according to any of aspects 1 to 4, wherein the cancer is selected from the group consisting of: breast cancer, prostate cancer, endometrial cancer, ovarian cancer, brain cancer, skin cancer, thyroid cancer, lung cancer, mesothelioma cancer, bladder cancer, pancreatic cancer, colorectal cancer, liver cancer, melanoma, glioblastoma, leukemia and lymphoma.
6. The method according to aspect 5, wherein the cancer is a lung cancer.
7. The method according to aspect 6, wherein the lung cancer is a non-small cell lung cancer or a lung adenocarcinoma.
8. The method according to any of aspects 6 or 7, wherein the lung cancer is a lung cancer with at least a mutation in the Epidermal Growth Factor Receptor (EGFR) gene causing resistance to tyrosine kinase inhibitors.
9. The method according to aspect 8, wherein the mutation in the EGFR is a deletion in the exon 19 or a L858R mutation in exon 21.
10. The method according to any one of aspects 1 to 9, wherein the sample is a tissue sample or a biofluid.
11. The method according to aspect 10, wherein the tissue sample is a sample from the tumor.
12. The method according to aspect 11, wherein the sample from the tumor is a lung cancer sample.
13. The method according to aspect 10, wherein the biofluid is blood, plasma or serum.
14. The method according to any one of aspects 1 to 13, wherein the expression level of BID is determined by measuring the BID mRNA levels or of a fragment thereof.
15. The method according to aspect 14, wherein the determination of the expression level of BID is carried out by RT-qPCR or nCounter.
16. A pharmaceutical composition comprising a TTKi, a BH3 mimetic agent and at least one pharmaceutically acceptable excipient.
17. The pharmaceutical composition according to aspect 16, wherein the TTKi is selected from the group consisting of: CFI-402257, AZ-3146, BAY1217389, BAY1161909, NMS-P715, TC Mps1 12, neversine, Mps1-IN-1, Mps1-IN-2, Mps1-IN-3, Mps BAY1, Mps BAY2a, Mps BAY2b, MPI-0479605, SP600125, S81694/NMS-P153; BOS172722; NTRC 0060-0 and NTRC 1501.
18. The pharmaceutical composition according to aspect 17, wherein the TTKi is BAY1217389.
19. The pharmaceutical composition according to aspect 16, wherein the BH3 mimetic agent is selected from the group consisting of: ABT-737, ABT-263, gossypol, obatoclax, venetoclax, SAHBA.
20. The pharmaceutical composition according to any one of aspects 16 to 19 for use in the treatment of cancer.
21. The pharmaceutical composition for use according to aspect 20, wherein the cancer is selected from the group consisting of: breast cancer, prostate cancer, endometrial cancer, ovarian cancer, brain cancer, skin cancer, thyroid cancer, lung cancer, mesothelioma cancer, bladder cancer, pancreatic cancer, colorectal cancer, liver cancer, melanoma, glioblastoma, leukemia and lymphoma.
22. The pharmaceutical composition for use according to any one of aspects 20 or 21, wherein the cancer is a lung cancer.
23. The pharmaceutical composition for use according to aspect 22, wherein the lung cancer is a non-small cell lung cancer or a lung adenocarcinoma.
24. The pharmaceutical composition for use according to any one of aspects 22 or 23, wherein the lung cancer is a lung cancer with at least a mutation in the Epidermal Growth Factor Receptor (EGFR) gene causing resistance to tyrosine kinase inhibitors.
25. The pharmaceutical composition for use according to aspect 24, wherein the mutation in the EGFR gene is a deletion in the exon 19 or a L858R mutation in exon 21.
26. Use of reagents specific for the determination of the expression level of BID for predicting the response of a patient suffering from cancer to a dual specificity protein kinase TTK inhibitor (TTKi) therapy or for designing a personalized therapy for a patient suffering cancer.
27. A method for predicting the response of a patient suffering from cancer to a TTKi therapy and treating the patient that comprises:
   a) determining the expression level of BH3-interacting domain death agonist (BID) in a sample from said patient; and
   b) comparing the expression level obtained in (i) to a reference value,
   wherein if the expression level of BID in the sample from the patient is above a reference value, said patient is treated with a TTKi.

The invention will be described by way of the following examples which are to be considered as merely illustrative and not limitative of the scope of the invention.

### EXAMPLES

### Material and methods

### Patients and tumor samples

The tumor samples used in the study derived from cancer patients diagnosed between 2015 and 2020 in three hospitals in Barcelona (Spain), Hospital Universitario Dexeus, Hospital de la Santa Creu i Sant Pau and Hospital Clinic de Barcelona. Studies were conducted in accordance with the Declaration of Helsinki, under an approved protocol of the Institutional Review Boards of the three participating hospitals (2020/122-ONC-DEX). Samples were de-identified for patient confidentiality and informed written consent was obtained from all subjects.

### Cell line culture and authentication

All parental cell lines used in the study were purchased from the ATCC or other cell banks, with the exception of PC9 and 11-18. Parental PC9 cells were provided by F. Hoffman-La Roche Ltd (Basel, Switzerland) with the authorization of Dr. Mayumi Ono (Kyushu University, Fukuoka, Japan); while parental 11-18 cells were kindly provided by Dr. Mayumi Ono. Resistant cells were derived by exposing parental cells to EGFR TKIs, as described in [Bertran-Alamillo et al., (2019) AURKB as a target in non-small cell lung cancer with acquired resistance to anti-EGFR therapy. Nat Commun 10, 1812, and Jacobsen K, et al., (2017) Convergent Akt activation drives acquired EGFR inhibitor resistance in lung cancer. Nat Commun 8, 410] and were maintained as polyclonal populations under concentrations of the appropriate drug ≥2.5 µM. All tissue culture reagents were acquired from Gibco/Thermo Fisher Scientific (Waltham, MA), unless otherwise specified. All tumor cells were grown in a humidified atmosphere with 5% CO2 at 37°C in RPMI 1640 + 10% fetal bovine serum (FBS), 50 µg/mL penicillin-streptomycin and 2 mM L-Glutamine. Cell lines were routinely tested for mycoplasma contamination and authenticated by analyzing >20 polymorphisms using a 30-gene panel NGS. In all cases, the genotypes and allelic fractions of parental and resistant cells were coincident.

### Cell viability analyses

Cells were seeded at a density of 2000-8000 per well in 96-well plates, allowed to attach for 24 h and treated with drugs for 2-3 doubling times (Table 1). LY3295668 and the rest of drugs were purchased from Selleck Chemicals (Houston, TX) or MedChem Express (Monmouth Junction, NJ). Each concentration of drug was tested in sextuplicates. After treatment, cells were incubated with medium containing 0.75 mg/mL of Thiazolyl Blue tetrazolium bromide (MTT, Alfa Aesar, Haverhill, MA) for 1-2 h at 37°C, medium was removed and formazan crystals dissolved in 100 µL DMSO. Cell numbers were estimated by measuring the absorbance at 565 nm, using an Infinite MPlex microplate reader (Tecan, Männedorf, Switzerland). Data were derived from at least two independent experiments. Combination experiments and calculation of combination indexes (CIs) were performed according to the Bliss method.

**Table 1. Sensitivity to TTK and AURKA inhibitors of tumor cell lines of different origins. In the two CNG-positive cell lines, the BID/MAPK1 ratio is indicated. GB, glioblastoma; LA, lung adenocarcinoma; LS, lung squamous cell carcinoma; Pr, prostate; UB, urinary bladder.**

| **Cell line** | **Origin** | **Td (h)** | **LY3295668 (AURKAi)** | | **BAY1217389 (TTKi)** | |
|---|---|---|---|---|---|---|
| | | | **IC50(µM)** | **%** | **IC50(µM)** | **%** |
| A549 | LA | 22 | **0.52 ± 0.07** | 50 ± 1 | **>1** | 55 ± 5 |
| DU-145 | Pr | 30 | **0.60 ± 0.12** | 51 ± 3 | **>1** | 54 ± 5 |
| EBC-1 | LS | 42 | **0.09 ± 0.02** | 39 ± 1 | **0.0046 ± 0.0006** | 29 ± 1 |
| FADU | HNSCC | 30 | **>10** | 68 ± 3 | **>1** | 73 ± 4 |
| HCC366 | LA | 65 | **0.22 ± 0.02** | 34 ± 2 | **>1** | 84 ± 3 |
| HCC78 | LA | 38 | **>10** | 74 ± 3 | **>1** | 52 ± 2 |
| LC-2/ad | LA | 58 | **0.83 ± 0.20** | 54 ± 2 | **0.0036 ± 0.0003** | 18 ± 1 |
| MDA-MB-231 | Breast | 25 | **>10** | 84 ± 1 | **>1** | 53 ± 2 |
| MDA-MB-468 | Breast | 35 | **0.05 ± 0.01** | 9 ± 0 | **0.0041 ± 0.0003** | 9 ± 1 |
| MIA-PaCa-2 | Pancreas | 26 | **>10** | 62 ± 8 | **>1** | 56 ± 5 |
| NCI-H1819 | LA | 51 | **0.45 ± 0.08** | 41 ± 2 | **0.0026 ± 0.0003** | 16 ± 0 |
| NCI-H1975 | LA | 39 | **0.40 ± 0.06** | 44 ± 0 | **>1** | 54 ± 4 |
| NCI-H2228 | LA | 96 | **0.07 ± 0.01** | 17 ± 1 | **>1** | 69 ± 0 |
| NCI-H23 | LA | 38 | **0.09 ± 0.01** | 38 ± 1 | **>1** | 64 ± 6 |
| NCI-H3122 | LA | 49 | **0.40 ± 0.08** | 37 ± 1 | **0.0072 ± 0.0005** | 21 ± 2 |
| RT-112 | UB | 24 | **1.57 ± 0.03** | 75 ± 2 | **0.0038 ± 0.0003** | 22 ± 0 |
| SK-MES-1 | LS | 50 | **0.10 ± 0.01** | 24 ± 1 | **>1** | 59 ± 9 |
| SK-OV-3 | Ovarian | 48 | **>10** | 59 ± 4 | **>1** | 64 ± 4 |
| SNU-C1 | Colon | 31 | **>10** | 72 ± 4 | **>1** | 67 ± 5 |
| T98G | GB | 22 | **>10** | 61 ± 4 | **>1** | 63 ± 4 |
| WM793 | Melan. | 48 | **>10** | 46 ± 0 | **>1** | 68 ± 9 |

### Cell-cycle analysis

For cell cycle analyses, cells were plated in T-25 flasks, allowed to attach, treated with drugs in RPMI 1640 + 10% FBS, trypsinised and centrifuged. Cell pellets were resuspended in PBS, fixed in 70% ethanol and incubated o/n at -20 °C. Fixed cells were subsequently re-centrifuged at 400 xg for 5 min at 4 °C; resuspended in 250 µL of PBS with 50 µg/mL RNAse A (Sigma Aldrich, Saint Louis, MO), incubated for 1 h at 37 °C and stained with propidium iodide (PI) (Roche Diagnostics, Basel, Switzerland) for 30 min at room temperature.

### Cell death assays

For cell death analyses, the Annexin-V-FLUOS (An) staining kit (Roche Diagnostics) was employed, according to the manufacturer's instructions. Stained cells were sorted with a FACSCanto II cytometer (BD Biosciences, Franklin Lakes, NJ) using the FACSDiva software version 6.1.2. The combination of Annexin V and PI was used to differentiate four cell populations; viable cells (An -/PI-), early apoptotic (An+/PI-), necrotic (An-/PI+) and later apoptotic/necrotic (An+/PI+).

### Western blot analysis

Cells were seeded in T-75 flasks, allowed to attach o/n and treated for 2-72 h in RPMI+10% FBS, unless otherwise indicated. Cultures were then washed twice with cold PBS, scrapped into RIPA buffer (Cell Signaling Technology, Danvers, MO) (20 mM Tris-HCl pH 7.5, 150 mM NaCl, 1 mM Na2EDTA, 1 mM EGTA, 1% NP-40, 1% sodium deoxycholate, 2.5 mM sodium pyrophosphate, 1 mM β-glycerophosphate, 1mM Na3VO4, 1 µg/ml leupeptin, 2 mM PMSF) with a protease inhibitors cocktail (Roche Diagnostics, Basel, Switzerland) and passed through an insulin syringe. Lysates were transferred to a microfuge tube, incubated on ice 15 minutes, centrifuged for 10 minutes at 14,000 rpm and immediately analyzed or frozen at -80 °C. After protein quantification using the Pierce BCA Protein Assay (Thermo Fisher Scientific), extracts (25 µg) were boiled in NuPAGE-LDS sample (Invitrogen, Carlsbad, CA) with 0.1% β-mercaptoethanol, resolved in 8% SDS-polyacrylamide gels and transferred to a PVDF membrane (Merck Millipore, Burlington, MA). Membranes were blocked for 1 h in Tris Buffered Saline (TBS, Bio-Rad, Hercules, CA) with 3% dry fat-free milk. After blocking, membranes were cut, incubated with primary antibodies (Supplementary Table 2) o/n at 4 °C, washed three times for 5 min each in PBS-Tween 0.1% (Bio-Rad) and incubated for 2 h with secondary antibody. Finally, after three additional washes, membranes were soaked in SuperSignal West Dura Chemiluminiscence substrate (Thermo Fisher Scientific, Waltham, MA) and read with a Bio-Rad ChemiDocMP Imaging System. ImageJ and ImageLab softwares were used quantify bands.

### DNA and RNA purification

In the case of FFPE tumor samples, 4 µm-slides were obtained by standard procedures and stained with hematoxylin and eosin. An expert pathologist determined the tumor area and evaluated the percentage of tumor infiltration. In samples with <20% tumor, manual macrodissection or laser capture microdissection (Zeiss, Jena, Germany) was used to ensure a minimum of 80% of tumor cells in the starting material for nucleic acid purification. RNA was extracted with a high purity FFPE RNA isolation kit (Roche, Mannheim, Germany), while the GeneRead DNA FFPE Kit or the QlAamp DNA FFPE Tissue Kit (Qiagen, Hilden, Germany) were used for DNA extraction, according to the manufacturer's instructions. For DNA and RNA purification from cultured cells, the DNeasy^{®} Blood & Tissue Kit (Qiagen) and the High Pure RNA isolation Kit (Roche Diagnostics) were employed. DNA utilized in WES was extracted from flash frozen cell pellets using the AllPrep DNA/RNA Micro Kit (Qiagen) and eluted in TE buffer, purity was determined by NanoDrop 8000 (Thermo Scientific) and DNA integrity was measured using a 4200 TapeStation (Agilent). All samples had a DIN of >7.0. In all cases, final concentrations of nucleic acids were measured by Qubit (Thermo Fisher Scientific).

### RT-Q-PCR and nCounter

Total RNA was converted into cDNA using the M-MLV reverse transcriptase enzyme. Quantification of gene expression was performed using a QuantStudio 6 Flex (Applied Biosystems). Template cDNA was added to Taqman Universal Master Mix (Applied Biosystems) in triplicate 12.5 µl reactions with specific primers and probes for each gene, as described. Primer and probe sets for BID and ACTB (reference gene) were designed using Primer Express 3.0 Software (Applied Biosystems) according to their Ref Seq (http://www.ensembl.org/index.html). Levels of mRNA were quantified according to the comparative ΔCt method using ACTB (β-actin) as endogenous gene. A sample was considered not evaluable and repeated when the standard deviation of the Ct values of the three replicates was > 0.30. nCounter was performed with the IO360 panel, according to the manufacturer's instructions (Nanostring, Seattle, WA). The 96 probes A and B of the custom panel were designed by Nanostring and are available upon request. Negative controls were used for background correction and the resulting counts were normalized using the housekeeping genes of the panel, as described in Aguado C, et al., 2020, Multiplex RNA-based detection of clinically relevant MET alterations in advanced non-small cell lung cancer, Mol Oncol 15, 350-363, and in Reguart N, et al., 2017, Identification of ALK, ROS1, and RET Fusions by a Multiplexed mRNA-Based Assay in Formalin-Fixed, Paraffin-Embedded Samples from Advanced Non-Small-Cell Lung Cancer Patients. Clin Chem 63, 751-760.

### Methods Combination Index

Cells were seeded at a density of 2000-8000 per well in 96-well plates, allowed to attach for 24 h and treated with drugs for 2-3 doubling times. The drugs were purchased from Selleckchem Chemicals (Houston, TX). Each concentration of drug was tested in six wells (technical replicates) in each experiment. After treatment, cells were incubated with medium containing 0.75 mg/mL of Thiazolyl Blue tetrazolium bromide (MTT, Thermo Fisher) for 1-2 h at 37°C, medium was removed and formazan crystals dissolved in 100 µL DMSO. Cell numbers were estimated by measuring the absorbance at 565 nm, using an Infinite MPlex microplate reader (Tecan, Männedorf, Switzerland). Combinations were tested in a minimum of two independent experiments performed on different days (biological replicates). Calculation of combination indexes (CIs) were performed according to the Bliss method.

### Statistical analysis

GraphPad Prism v6.0 was used for all statistical analyses; statistical tests are indicated in the detailed description of drawings. Two-group comparisons were made using unpaired two-tailed Student's t tests, with Welch's correction when variances were significantly different according to an F test. In the case of xenografts, two-way ANOVA was employed for comparisons in tumor sizes and Mann-Whitney U test for statistical fold-change in tumor volumes. Data are presented as mean ± SEM or SD, as indicated; p-values of <0.05 were considered to be statistically significant.

### Results

The authors of the present invention have used BAY1217389, a highly specific TTKi currently in Phase I trials, finding six clones sensitive to the TTKi showing IC50s < 1 nM the twelve TTKi resistant clones presenting IC50s > 200 nM. In contrast, the AURKA inhibitor LY3295668 and the DNA-damaging agent cisplatin showed similar efficacy in the 18 clones (Fig. 1A, Fig. 1B). Flow cytometry revealed that, after G2/M arrest, BAY1217389 triggered extensive polyploidy in resistant clones but cell death in sensitive ones (Figs. 1C-D).

Western blotting and RNA analysis of BID revealed upregulation exclusively in clones sensitive to TTKi (Fig. 2A-B-C). Dose-response curves revealed a shift to complete resistance to BAY1287389 in PC9-ER after BID gene silencing, with IC50s increasing to >10 µM.

The experiments presented so far established the association of BID expression with response to TTKi in EGFR-mut, NSCLC cells. Next, inventors decided to investigate tumor cells of other origins and genetic backgrounds. To this end, they selected 19 lines derived from lung, breast, pancreatic, bladder, prostate, breast, ovarian, head and neck and brain carcinomas and determined the dose-response curves to BAY1217389 (Fig. 3A). Five of the 19 cell lines were sensitive to TTKi; experiencing extensive cell death under visual inspection and showing IC50s < 7nM for BAY1217389. Two of the sensitive cell lines were fusion-positive lung adenocarcinoma cell lines; the remaining three were of breast, bladder and lung origin and did not harbor known oncogenic drivers. In contrast, 14 cell lines were resistant to TTKi, with IC50s> 1 µM and >50% survival even at high concentrations of drug.

The five cell lines sensitive to BAY1217389 expressed significantly higher levels of BID mRNA and protein than the rest of the panel. In fact, among the 6 lines with high BID mRNA (2-ΔCtx100 ≥ 0.5) and protein, only HCC78 cells were not sensitive to TTKi; while the 13 cell lines with low BID were uniformly resistant (Fig. 3B-E). In contrast, BID expression did not correlate with IC50s for LY3295668 (Fig. 3F). Remarkably, when inventors plotted the BID mRNA levels vs. the growth inhibition by BAY1217389 of all the EGFR-wt cell lines and EGFR-mut clones included in their study, a saturation kinetics was observed (r2=0.58) (Fig. 3G). Flow cytometry of the TTKi sensitive NCl-H1819 cells revealed that BAY1217389 triggered G2/M arrest, while Annexin V indicated subsequent, extensive induction of cell death (Fig. 3H-I).

To further confirm that BID is a general regulator of response to TTKi in tumor cells, inventors used BID silencing with shRNA lentiviruses in the TTKi-sensitive NCI-H1819 (Fig. 4A-B). They found that BID silencing rendered NCI-H1819 cells resistant to TTKi, with no effects on response to AURKAi.

Regarding BID upregulation in human cancer, COSMIC reports 445/9087 (5%) tumor samples as BID overexpressors. Frequencies in the most common malignancies range between 4 and 6%, being higher in kidney, urinary tract, stomach, and cervix tumors (Fig. 5A). BID upregulation significantly associates with TP53 mutations but shows a trend of mutual exclusivity with KRAS or EGFR driver mutations, which reaches statistical significance in the case of BRAF (Fig. 5B).

To validate the COSMIC data, inventors performed a prevalence study of BID upregulation in samples from their institutions. First, they re-analyzed the results previously obtained in 67 FFPE Stage IIIB-IV lung cancer samples previously tested with a 770-gene expression panel that includes BID. When using the geomean plus two times the standard deviation (geomean+2×SD) as a cut-off value, four tumors (6.2%) showed BID mRNA upregulation (Fig. 5C). Then, they prospectively tested by RT-Q-PCR a total of 96 FFPE tumor samples of different histologies, together with FFPE blocks of 11 cell lines of known BID status. Using the geomean+2×SD as a cut-off, 6/96 (6.3%) samples presented BID upregulation. Remarkably, this cut-off value (0.34) was very similar to the BID mRNA levels in FFPE blocks of the lowest-expressing cell lines sensitive to SAC abrogation (Fig. 5D).

## Claims

1. An *in vitro* method for predicting the response of a patient suffering cancer to a dual specificity protein kinase TTK inhibitor (TTKi) therapy that comprises:
i) determining the expression level of BH3-interacting domain death agonist (BID) in a sample from said patient and,
ii) comparing the expression level obtained in (i) to a reference value,
wherein if the expression level of BID in a sample from the patient is above a reference value, it indicates that the patient is predicted to respond to the TTKi therapy.

2. An *in vitro* method for designing a personalized therapy for a patient suffering cancer that comprises:
i) determining the expression level of BH3-interacting domain death agonist (BID) in a sample from said patient and,
ii) comparing the expression level obtained in (i) to a reference value,
wherein an expression level of BID above a reference value is indicative that the patient is to be treated with a TTKi.

3. The method according to any one of claims 1 or 2, wherein the TTKi is selected from the group consisting of: CFI-402257, AZ-3146, BAY1217389, BAY1161909, NMS-P715, TC Mps1 12, reversine, Mpsl-IN-1, Mpsl-IN-2, Mpsl-IN-3, Mps BAY1, Mps BAY2a, Mps BAY2b, MPI-0479605, S81694/NMS-P153; BOS172722; NTRC 0060-0 and NTRC 1501, preferably is BAY1217389, BOS172722 or CFI-402257.

4. The method according to any one of claims 1 to 3, wherein the cancer is a lung cancer, preferably a non-small cell lung cancer or a lung adenocarcinoma.

5. The method according to claim 4, wherein the lung cancer is a lung cancer with at least a mutation in the Epidermal Growth Factor Receptor (EGFR) gene causing resistance to tyrosine kinase inhibitors, preferably is a deletion in the exon 19 or a L858R mutation in exon 21.

6. The method according to any one of claims 1 to 5, wherein the sample is a tissue sample, preferably a sample from the tumor, more preferably a lung cancer sample, or a biofluid, preferably blood, plasma or serum.

7. The method according to any one of claims 1 to 6, wherein the expression level of BID is determined by measuring the BID mRNA levels or of a fragment thereof.

8. A pharmaceutical composition comprising a TTKi, a BH3 mimetic agent and at least one pharmaceutically acceptable excipient.

9. The pharmaceutical composition according to claim 8, wherein the TTKi is BAY1217389.

10. The pharmaceutical composition according to claim 8, wherein the BH3 mimetic agent is selected from the group consisting of: ABT-737, ABT-263, gossypol, obatoclax, venetoclax, SAHB_{A}.

11. The pharmaceutical composition according to any one of claims 8 to 10 for use in the treatment of cancer.

12. The pharmaceutical composition for use according to any one of claim 11, wherein the cancer is a lung cancer, preferably is a non-small cell lung cancer or a lung adenocarcinoma.

13. The pharmaceutical composition for use according to any one of claims 11 or 12, wherein the lung cancer is a lung cancer with at least a mutation in the Epidermal Growth Factor Receptor (*EGFR*) gene causing resistance to tyrosine kinase inhibitors, preferably is a deletion in the exon 19 or a L858R mutation in exon 21.

14. Use of reagents specific for the determination of the expression level of BID for predicting the response of a patient suffering from cancer to a dual specificity protein kinase TTK inhibitor (TTKi) therapy or for designing a personalized therapy for a patient suffering cancer.

15. A method for predicting the response of a patient suffering from cancer to a TTKi therapy and treating the patient that comprises:
a) determining the expression level of BH3-interacting domain death agonist (BID) in a sample from said patient; and
b) comparing the expression level obtained in (i) to a reference value,
wherein if the expression level of BID in the sample from the patient is above a reference value, said patient is treated with a TTKi.
